# EUROPÄISCHE PATENTSCHRIFT

(11) **EP 1 966 161 B1**
(45) Veröffentlichungstag und Bekanntmachung des Hinweises auf die Patenterteilung: **28.09.2011**
(21) Anmeldenummer: 06829297.8
(22) Anmeldetag: 05.12.2006
(51) Int. Cl.: C07D 239/42, C07D 417/12, C07D 403/12, C07D 409/12, A01N 43/54, A01N 43/78

(54) **CARBOXAMIDE ZUR BEKÄMPFUNG UNERWÜNSCHTER MIKROORGANISMEN IM PFLANZENSCHUTZ**
CARBOXAMIDES FOR CONTROLLING UNDESIRED MICRO-ORGANISMS IN PLANT PROTECTION
CARBOXAMIDES POUR LUTTER CONTRE LES MICRO-ORGANISMES INDESIRABLES DANS LA PROTECTION PHYTOSANITAIRE

(30) Priorität: 17.12.2005 DE 102005060449
(43) Veröffentlichungstag der Anmeldung: 10.09.2008
(73) Patentinhaber: Bayer CropScience AG, 40789 Monheim (DE)
(72) Erfinder: DUNKEL, Ralf, F-69001 Lyon (FR); ELBE, Hans-Ludwig, 42329 Wuppertal (DE); GREUL, Jörg, Nico, 42799 Leichlingen (DE); GAYER, Herbert, 40789 Monheim (DE); DAHMEN, Peter, 41470 Neuss (DE); WACHENDORFF-NEUMANN, Ulrike, 56566 Neuwied (DE); VOERSTE, Arnd, 50674 Köln (DE)
(86) Internationale Anmeldenummer: PCT/EP2006/011651
(87) Internationale Veröffentlichungsnummer: WO 2007/068375

(56) Entgegenhaltungen:
- EP-A- 0 545 099
- WO-A-03/070705

## Beschreibung

Die vorliegende Erfindung betrifft neue Carboxamide, mehrere Verfahren zu deren Herstellung und deren Verwendung zur Bekämpfung von schädlichen Mikroorganismen im Pflanzenschutz und Materialschutz.

Es ist bereits bekannt geworden, dass zahlreiche Carboxamide fungizide Eigenschaften besitzen (vgl. z.B. WO 03/070705, EP-A 0 545 099 und JP-A 9-132567). Die Wirksamkeit der dort beschriebenen Stoffe ist gut, lässt aber in manchen Fallen zu wünschen übrig.

Es wurden nun neue Carboxamide der Formel (I) 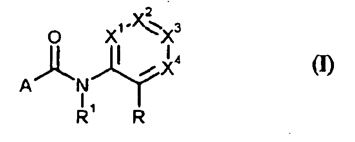 gefunden, in welcher
- R: für gegebenenfalls einfach bis fünffach durch W¹ substituiertes Phenyl oder die Gruppierung 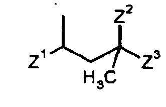 steht,
- W¹: für Halogen, Cyano, Nitro, C₁-C₆-Alkyl, C₁-C₄-Alkoxy, C₁-C₄-Alkylthio, C₁-C₄-Alkylsulfo- nyl, C₂-C₆-Alkenyl, C₃-C₆-Cycloalkyl oder für C₁-C₆-Halogenalkyl, C₁-C₆-Halogenalkoxy, C₁-C₆-Halogenalkylthio oder C₁-C₆-Halogenalkylsulfonyl mit jeweils 1 bis 13 Halogen- atomen, oder für -C(Q²)=N-Q³- steht, worin
Q² für Wasserstoff, Hydroxy, C₁-C₄-Alkyl, C₁-C₄-Halogenalkyl 1 bis 9 Halogenatomen oder C₃-C₆-Cycloalkyl steht,
Q³ für Hydroxy, Amino, Methylamino, Phenyl, Benzyl oder für jeweils gegebenenfalls durch Halogen, Cyano, Hydroxy, C₁-C₄-Alkoxy, C₁-C₄-Alkylthio, C₁-C₄-Alkyl- amino, Di(C₁-C₄-alkyl)amino oder Phenyl substituiertes C₁-C₄-Alkyl oder C₁-C₄- Alkoxy, oder für C₂-C₄-Alkenyloxy oder C₂-C₄-Alkinyloxy steht,
- Z¹: für Wasserstoff oder Methyl steht,
- Z²: für Wasserstoff oder Methyl steht,
- Z³: für Methyl oder Ethyl steht,
- R¹: für Wasserstoff, C₁-C₈-Alkyl, C₁-C₆-Alkylsulfinyl, C₁-C₆-Alkylsulfonyl, C₁-C₄-Alkoxy-C₁- C₄-alkyl, C₃-C₈-Cycloalkyl; C₁-C₆-Halogenalkyl, C₁-C₄-Halogenalkylthio, C₁-C₄-Halogenal- kylsulfinyl, C₁-C₄-Halogenalkylsulfonyl, Halogen-C₁-C₄-alkoxy-C₁-C₄-alkyl, C₃-C₈-Halo- gencycloalkyl mit jeweils 1 bis 9 Fluor-, Chlor- und/oder Bromatomen; Formyl, Formyl-C₁- C₃-alkyl, (C₁-C₃-Alkyl)carbonyl-C₁-C₃-alkyl, (C₁-C₃-Alkoxy)carbonyl-C₁-C₃-alkyl; Halo- gen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen;
(C₁-C₈-Alkyl)carbonyl, (C₁-C₈-Alkoxy)carbonyl, (C₁-C₈-Alkylthio)carbonyl, (C₁-C₄-Alkoxy- C₁-C₄-alkyl)carbonyl, (C₃-C₆-Alkenyloxy)carbonyl, (C₃-C₆-Alkinyloxy)carbonyl, (C₃-C₈- Cycloalkyl)carbonyl; (C₁-C₆-Halogenalkyl)carbonyl, (C₁-C₆-Halogenalkoxy)carbonyl, (C₁- C₆-Halogenalkylthio)carbonyl, (Halogen-C₁-C₄-alkoxy-C₁-C₄-alkyl)carbonyl, (C₃-C₆-Halo- genalkenyloxy)carbonyl, (C₃-C₆-Halogenalkinyloxy)carbonyl, (C₃-C₈-Halogencycloalkyl)- carbonyl mit jeweils 1 bis 9 Fluor-, Chlor- und/oder Bromatomen; oder -CH₂-C≡C-R^{1-A}, -CH₂-CH=CH-R^{1-A}, -CH=C=CH-R^{1-A}, -C(=O)C(=O)R², -CONR³R⁴ oder -CH₂NR⁵R⁶ steht,
- R^{1-A}: für Wasserstoff, C₁-C₆-Alkyl, C₁-C₆-Halogenalkyl, C₂-C₆-Alkenyl, C₂-C₆-Alkinyl, C₃-C₇- Cycloalkyl, (C₁-C₄-Alkoxy)carbonyl, (C₃-C₆-Alkenyloxy)carbonyl, (C₃-C₆-Alkinyloxy)- carbonyl oder Cyano steht,
- R²: für Wasserstoff, C₁-C₈-Alkyl), C₁-C₈-Alkoxy, C₁-C₄-Alkoxy-C₁-C₄-alkyl, C₃-C₈-Cycloalkyl; C₁-C₆-Halogenalkyl, C₁-C₆-Halogenalkoxy, Halogen-C₁-C₄-alkoxy-C₁-C₄-alkyl, C₃-C₈-Halo- gencycloalkyl mit jeweils 1 bis 9 Fluor-, Chlor- und/oder Bromatomen steht,
- R³ und R⁴: unabhängig voneinander jeweils für Wasserstoff, C₁-C₈-Alkyl, C₁-C₄-Alkoxy-C₁-C₄-alkyl, C₃-C₈-Cycloalkyl; C₁-C₈-Halogenalkyl, Halogen-C₁-C₄-alkoxy-C₁-C₄-alkyl C₃-C₈-Halogen- cycloalkyl mit jeweils 1 bis 9 Fluor-, Chlor- und/oder Bromatomen stehen,
- R³ und R⁴: außerdem gemeinsam mit dem Stickstoffatom, an das sie gebunden sind, einen gegebenen- falls einfach oder mehrfach, gleich oder verschieden durch Halogen oder C₁-C₄-Alkyl substituierten gesättigten Heterocyclus mit 5 bis 8 Ringatomen bilden, wobei der Hetero- cyclus 1 oder 2 weitere, nicht benachbarte Heteroatome aus der Reihe Sauerstoff, Schwefel oder NR⁷ enthalten kann,
- R⁵und R⁶: unabhängig voneinander für Wasserstoff, C₁-C₈-Alkyl, C₃-C₈-Cycloalkyl; C₁-C₈-Halogenalkyl, C₃-C₈-Halogencycloalkyl mit jeweils 1 bis 9 Fluor-, Chlor- und/oder Bromatomen stehen,
- R⁵ und R⁶: außerdem gemeinsam mit dem Stickstoffatom, an das sie gebunden sind, einen gegebenen- falls einfach oder mehrfach, gleich oder verschieden durch Halogen oder C₁-C₄-Alkyl sub- stituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR⁷ enthalten kann,
- R⁷: für Wasserstoff oder C₁-C₆-Alkyl steht,
- X¹, X², X³ und X⁴: unabhängig voneinander für N oder CR⁸ stehen mit der Maßgabe, dass wenigstens einer dieser Reste für N steht,
- R⁸: für Wasserstoff, Fluor, Chlor, Methyl, iso-Propyl, Methylthio oder Trifluormethyl steht,
- A: für einen der folgenden Reste A1 bis A18 steht 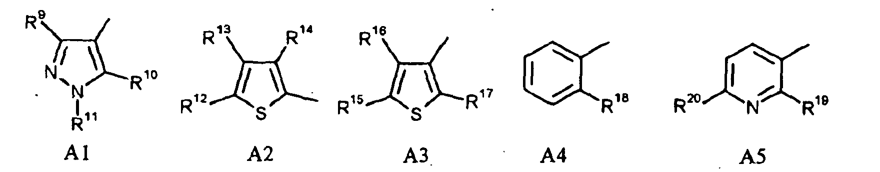 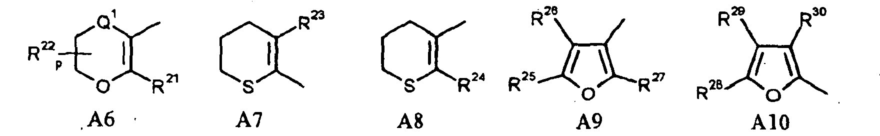 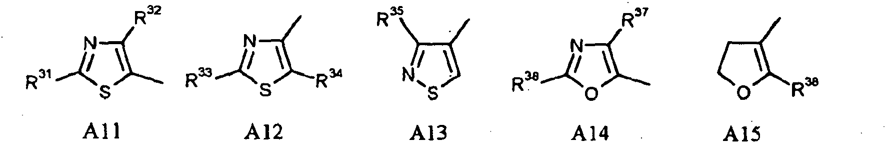 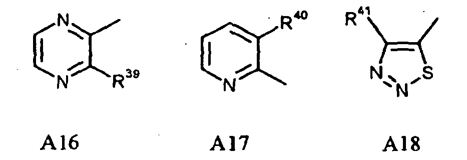
- R⁹: für Wasserstoff, Cyano, Halogen, Nitro, C₁-C₄-Alkyl, C₁-C₄-Alkoxy, C₁-C₄-Alkylthio, C₃- C₆-Cycloalkyl, C₁-C₄-Halogenalkyl, C₁-C₄-Halogenalkoxy oder C₁-C₄-Halogenalkylthio mit jeweils 1 bis 5 Halogenatomen, Aminocarbonyl oder Aminocarbonyl-C₁-C₄-alkyl steht,
- R¹⁰: für Wasserstoff, Halogen, Cyano, C₁-C₄-Alkyl, C₁-C₄-Alkoxy oder C₁-C₄-Alkylthio steht,
- R¹¹: für Wasserstoff, C₁-C₄-Alkyl, Hydroxy-C₁-C₄-alkyl, C₂-C₆-alkenyl, C₃-C₆-Cycloalkyl, C₁-C₄- Alkylthio-C₁-C₄-alkyl, C₁-C₄-Alkoxy-C₁-C₄-alkyl, C₁-C₄-Halogenalkyl, C₁-C₄-Halogenalkylthio- C₁-C₄-alkyl, C₁-C₄-Halogenalkoxy-C₁-C₄-alkyl mit jeweils 1 bis 5 Halogenatomen, Phenyl, (C₁- C₄-Alkyl)carbonyl, (C₁-C₄-Alkoxy)carbonyl, (C₁-C₄-Alkylthio)carbonyl, (C₁-C₄-Alkoxy-C₁- C₄-alkyl)carbonyl; (C₁-C₄-Halogenalkyl)carbonyl, (C₁-C₄-Halogenalkoxy)carbonyl, (C₁-C₄- Halogenalkylthio)carbonyl, (Halogen-C₁-C₄-alkoxy-C₁-C₄-alkyl)carbonyl mit jeweils 1 bis 9 Halogenatomen steht,
- R¹² und R¹³: unabhängig voneinander für Wasserstoff, Halogen, C₁-C₄-Akyl oder C₁-C₄-Halogen- alkyl mit 1 bis 5 Halogenatomen steht,
- R¹⁴: für Halogen, Cyano oder C₁-C₄-Alkyl, oder C₁-C₄-Halogenalkyl oder C₁-C₄-Halogenalkoxy mit jeweils 1 bis 5 Halogenatomen steht,
- R¹⁵ und R¹⁶: unabhängig voneinander für Wasserstoff, Halogen, C₁-C₄-Alkyl oder C₁-C₄- Halogenalkyl mit 1 bis 5 Halogenatomen steht,
- R¹⁷: für Wasserstoff, C₁-C₄-Alkyl oder C₁-C₄-Halogenalkyl mit 1 bis 5 Halogenatomen steht,
- R¹⁸: für Wasserstoff, Halogen, Hydroxy, Cyano, C₁-C₆-Alkyl, C₁-C₄-Halogenalkyl, C₁-C₄-Ha- logenalkoxy oder C₁-C₄-Halogenalkylthio mit jeweils 1 bis 5 Halogenatomen steht,
- R¹⁹: für Halogen, Hydroxy, Cyano, C₁-C₄-Alkyl, C₁-C₄-Alkoxy, C₁-C₄-Alkylthio, C₁-C₄- Halogenalkyl, C₁-C₄-Halogenalkylthio oder C₁-C₄-Halogenalkoxy mit jeweils 1 bis 5 Halogenatomen steht,
- R²⁰: für Wasserstoff, Halogen, Cyano, C₁-C₄-Alkyl, C₁-C₄-Alkoxy, C₁-C₄-Alkylthio, C₁-C₄-Halo- genalkyl, C₁-C₄-Halogenalkoxy mit jeweils 1 bis 5 Halogenatomen, C₁-C₄-Alkylsulphinyl oder C₁-C₄-Alkylsulphonyl steht,
- R²¹: für C₁-C₄-Alkyl oder C₁-C₄-Halogenalkyl mit 1 bis 5 Halogenatomen steht,
- R²²: für C₁-C₄-Alkyl steht,
- Q¹: für S (Schwefel), SO, SO₂ oder CH₂ steht,
- p: für 0, 1 oder 2, wobei R²² für identische oder verschiedene Reste steht, wenn p für 2 steht,
- R²³: für C₁-C₄-Alkyl oder C₁-C₄-Halogenalkyl mit 1 bis 5 Halogenatomen steht,
- R²⁴: für C₁-C₄-Alkyl oder C₁-C₄-Halogenalkyl mit 1 bis 5 Halogenatomen steht,
- R²⁵ und R²⁶: unabhängig voneinander für Wasserstoff, Halogen, Amino, C₁-C₄-Alkyl oder C₁-C₄- Halogenalkyl mit 1 bis 5 Halogenatomen stehen,
- R²⁷: für Wasserstoff, C₁-C₄-Alkyl oder C₁-C₄-Halogenalkyl mit 1 bis 5 Halogenatomen steht,
- R²⁸ und R²⁹: unabhängig voneinander für Wasserstoff, Halogen, Amino, Nitro, C₁-C₄-Alkyl oder C₁- C₄-Halogenalkyl mit 1 bis 5 Halogenatomen stehen,
- R³⁰: für Wasserstoff, Halogen, C₁-C₄-Alkyl oder C₁-C₄-Halogenalkyl mit 1 bis 5 Halogenatomen steht,
- R³¹: für Wasserstoff, Halogen, Amino, C₁-C₄-Akylamino, Di-(C₁-C₄-alkyl)amino, Cyano, C₁-C₄- Alkyl oder C₁-C₄-Halogenalkyl mit 1 bis 5 Halogenatomen steht,
- R³²: für Halogen, C₁-C₄-Alkyl oder C₁-C₄-Halogenalkyl mit 1 bis 5 Halogenatomen steht,
- R³³: für Wasserstoff, Halogen, Amino, C₁-C₄-Alkylamino, Di-(C₁-C₄-alkyl)amino, Cyano, C₁-C₄- Alkyl oder C₁-C₄-Halogenalkyl mit 1 bis 5 Halogenatomen steht,
- R³⁴: für Halogen, C₁-C₄-Alkyl oder C₁-C₄-Halogenalkyl mit 1 bis 5 Halogenatomen steht,
- R³⁵: für Halogen, C₁-C₄-Alkyl oder C₁-C₄-Halogenalkyl mit 1 bis 5 Halogenatomen steht,
- R³⁶: für Wasserstoff oder C₁-C₄-Alkyl steht,
- R³¹: für Halogen oder C₁-C₄-Alkyl steht,
- R³⁸: für C₁-C₄-Alkyl oder C₁-C₄-Halogenalkyl mit 1 bis 5 Halogenatomen steht,
- R³⁹: für Wasserstoff, Halogen, C₁-C₄-Alkyl oder C₁-C₄-Halogenalkyl mit 1 bis 5 Halogenatomen steht,
- R⁴⁰: für Halogen, Hydroxy, C₁-C₄-Alkyl, C₁-C₄-Alkoxy, C₁-C₄-Alkylthio, C₁-C₄-Halogenalkyl, C₁-C₄-Halogenalkylthio oder C₁-C₄-Halogenalkoxy mit jeweils 1 bis 5 Halogenatomen steht,
- R⁴¹: für C₁-C₄-Alkyl steht.

Weiterhin wurde gefunden, dass man Carboxamide der Formel (I) erhält, indem man
(a) Carbonsäurehalogenide der Formel (II) 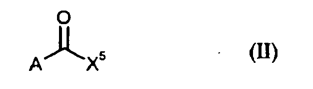 in welcher
   - A: die oben angegebenen Bedeutungen hat,
   - X⁵: für Halogen oder Hydroxy steht,
   mit Anilinderivaten der Formel (III) 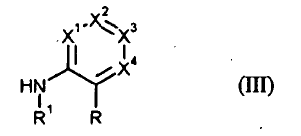 in welcher R, R¹, X¹, X², X³ und X⁴ die oben angegebenen Bedeutungen haben, gegebenenfalls in Gegenwart eines Kupplungsreagenzes, gegebenenfalls in Gegenwart eines Säurebindemittels und gegebenenfalls in Gegenwart eines Verdünnungsmittels umsetzt,
   oder
(b) Carboxamide der Formel (I-a) 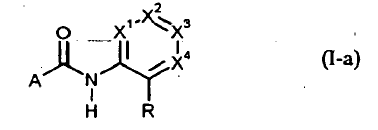 in welcher R, X¹, X², X³, X⁴ und A die oben angegebenen Bedeutungen haben, mit Halogeniden der Formel (IV)

   R^{1-B}-Hal (IV)

   in welcher
   - R^{1-B}: für C₁-C₈-Alkyl, C₁-C₆-Alkylsulfinyl, C₁-C₆-Alkylsulfonyl, C₁-C₄-Alkoxy-C₁-C₄-al- kyl, C₃-C₈-Cycloalkyl; C₁-C₆-Halogenalkyl, C₁-C₄-Halogenalkylthio, C₁-C₄-Halo- genalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)- carbonyl-C₁-C₃-alkyl; Halogen-(C₁-C₃-alkyl)carbonyl-C₁-C₃-alkyl, Halogen-(C₁-C₃- alkoxy)carbonyl-C₁-C₃-alkyl mit jeweils 1 bis 13 Fluor-, Chlor- und/oder Bromatomen;
   (C₁-C₈-Alkyl)carbonyl, (C₁-C₈-Alkoxy)carbonyl, (C₁-C₈-Alkylthio)carbonyl, (C₁-C₄- Alkoxy-C₁-C₄-alkyl)carbonyl, (C₃-C₆-Alkenyloxy)carbonyl, (C₃-C₆-Alkinyloxy)car- bonyl, (C₃-C₈-Cycloalkyl)carbonyl; (C₁-C₆-Halogenalkyl)carbonyl, (C₁-C₆-Halogen- alkoxy)carbonyl, (C₁-C₆-Halogenalkylthio)carbonyl, (Halogen-C₁-C₄-alkoxy-C₁-C₄- alkyl)carbonyl, (C₃-C₆-Halogenalkenyloxy)carbonyl, (C₃-C₆-Halogenalkinyloxy)car- bonyl, (C₃-C₈-Halogencycloalkyl)carbonyl mit jeweils 1 bis 9 Fluor-, Chlor- und/oder Bromatomen; oder -CH₂-C≡C-R^{1-A} -CH₂-CH=CH-R^{1-A}, -CH=C=CH-R^{1-A}, -C(=O)C(=O)R², -CONR³R⁴ oder -CH₂NR⁵R⁶ steht,
   R^{1-A}, R², R³, R⁴, R⁵ und R⁶ die oben angegebenen Bedeutungen haben,
   - Hal: für Chlor, Brom oder Iod steht,
   in Gegenwart einer Base und in Gegenwart eines Verdünnungsmittels umsetzt.

Schließlich wurde gefunden, dass die neuen Carboxamide der Formel (I) sehr gute mikrobizide Eigenschaften besitzen und zur Bekämpfung unerwünschter Mikroorganismen sowohl im Pflanzenschutz als auch im Materialschutz verwendbar sind.

Die erfindungsgemäßen Verbindungen können gegebenenfalls als Mischungen verschiedener möglicher isomerer Formen, insbesondere von Stereoisomeren, wie z. B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die erfindungsgemäßen Carboxamide sind durch die Formel (I) allgemein definiert. Bevorzugte Restedefinitionen der vorstehenden und nachfolgend genannten Formeln sind im Folgenden angegeben. Diese Definitionen gelten für die Endprodukte der Formel (I) wie für alle Zwischenprodukte gleichermaßen.
- R: steht bevorzugt für einfach substituiertes Phenyl, wobei die Substituenten aus der Liste W¹ ausgewählt sind.
- R: steht auch bevorzugt für zweifach, gleich oder verschieden substituiertes Phenyl, wobei die Substituenten aus der Liste W¹ ausgewählt sind.
- R: steht auch bevorzugt für dreifach, gleich oder verschieden substituiertes Phenyl, wobei die Substituenten aus der Liste W¹ ausgewählt sind.
- R: steht besonders bevorzugt für einfach in 4-Position substituiertes Phenyl, wobei die Substitu- enten aus der Liste W¹ ausgewählt sind.
- R: steht besonders bevorzugt für zweifach, gleich oder verschieden in 3,4-Position substituiertes Phenyl, wobei die Substituenten aus der Liste W¹ ausgewählt sind.
- R: steht besonders bevorzugt für zweifach, gleich oder verschieden in 2,4-Position substituiertes Phenyl, wobei die Substituenten aus der Liste W¹ ausgewählt sind.
- R: steht besonders bevorzugt für zweifach, gleich oder verschieden in 3,5-Position substituiertes Phenyl, wobei die Substituenten aus der Liste W¹ ausgewählt sind.
- R: steht besonders bevorzugt für dreifach, gleich oder verschieden in 2,4,6-Position substituiertes Phenyl, wobei die Substituenten aus der Liste W¹ ausgewählt sind.
- R: steht auch bevorzugt für eine der folgenden Gruppierungen Z1 bis Z8 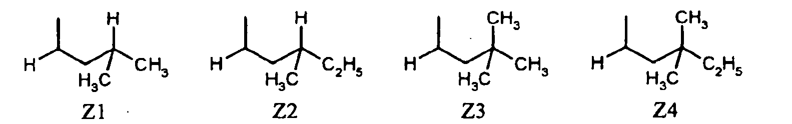 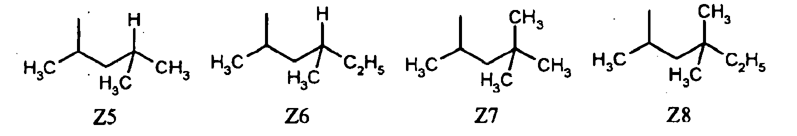
- R: steht auch besonders bevorzugt für Z1, Z3, Z5 oder Z7.
- R: steht auch ganz besonders bevorzugt für Z1.
- R: steht auch ganz besonders bevorzugt, für Z3.
- R: steht auch ganz besonders bevorzugt für Z5.
- R: steht auch ganz besonders bevorzugt für Z7.
- W¹: steht bevorzugt für Fluor, Chlor, Brom, Iod, Cyano, Nitro, C₁-C₄-Alkyl, C₁-C₄-Alkoxy, C₁- C₄-Alkylthio, C₁-C₄-Alkylsulfonyl, C₂-C₄-Alkenyl, C₃-C₆-Cycloalkyl oder für C₁-C₄- Halogenalkyl, C₁-C₄-Halogenalkoxy, C₁-C₄-Halogenalkylthio oder C₁-C₄-Halogenalkyl- sulfonyl mit jeweils 1 bis 9 Fluor-, Chlor- oder Bromatomen oder für -C(Q²)=N-Q³, worin
Q² für Wasserstoff, Methyl, Ethyl, Trifluormethyl oder Cyclopropyl steht und
Q³ für Hydroxy, Methoxy, Ethoxy, Propoxy oder Isopropoxy steht.
- W¹: steht besonders bevorzugt für Fluor, Chlor, Brom, Iod, Methyl, Ethyl, n-, iso-Propyl, n-, i-, s-, t- Butyl, Methoxy, Ethoxy, n-, iso-Propoxy, n-, i-, s-, t-Butoxy, Methylthio, Ethylthio, n-, iso-Pro- pylthio, n-, i-, s-, t-Butylthio, Methylsulfonyl, Ethylsulfonyl, n-, iso-Propylsulfonyl, n-, i-, s-, t- Butylsulfonyl, Allyl, Cyclopropyl, Cyclobutyl, Cyclopentyl, Cyclohexyl, Monofluormethyl, Mo- nofluorethyl, Difluormethyl, Trifluormethyl, Difluorchlormethyl, Trichlormethyl, Dichlormethyl, Monofluormethoxy, Monofluorethoxy, Difluormethoxy, Trifluormethoxy, Difluorchlormethoxy, Trichlormethoxy, Dichlormethoxy, Monofluormethylthio, Monofluorethylthio, Difluormethylthio, Trifluormethylthio, Difluorchlormethylthio, Trichlormethylthio, Dichlormethylthio, Monofluormethylsulfonyl, Monofluorethylsulfonyl, Difluormethylsulfonyl, Trifluorme- thylsulfonyl, Difluorchlormethylsulfonyl, Trichlormethylsulfonyl, Dichlormethylsulfonyl, -CH=N-OH, -CH=N-OCH₃, -CH=N-OC₂H₅, -CH=N-O(n-C₃H₇), -CH=N-O(i-C₃H₇), -C(CH₃)=N-OH, -C(CH₃)=N-OCH₃, -C(CH₃)=N-OC₂H₅, -C(CH₃)=N-O(n-C₃H₇), -C(CH₃)=N-O(i-C₃H₇), -C(C₂H₅)=N-OH, -C(C₂H₅)=N-OCH₃, -C(C₂H₅)=N-OC₂H₅, -C(C₂H₅)=N-O(n-C₃H₇), -C(C₂H₅)=N-O(i-C₃H₇), -C(CF₃)=N-OH, -C(CF₃)=N-OCH₃, -C(CF₃)N-OC₂H₅, -C(CF₃)=N-O(n-C₃H₇), -C(CF₃)=N-O(i-C₃H₇), -C(Cyclopropyl)=N-OH, -C(Cyclopropyl)=N-OCH₃, -C(Cyclopropyl)=N-OC₂H₅, -C(Cyclopropyl)=N-O(n-C₃H₇), -C(Cyclopropyl)=N-O(i-C₃H₇).
- W¹: steht ganz besonders bevorzugt für Fluor, Chlor, Brom, Methyl, Methoxy, Methylthio, Methylsulfonyl, Difluormethyl, Trifluormethyl, Trichlormethyl, Dichlormethyl, Trifluor- methoxy, Trichlormethoxy, Trifluormethylthio, Trichlormethylthio, Trifluormethylsulfonyl, -C(CH₃)=N-OCH₃, -C(CH₃)=N-O(i-C₃H₇), -C(Cyclopropyl)=N-OCH₃.
- R¹: steht bevorzugt für Wasserstoff, C₁-C₆-Alkyl, C₁-C₄-Akylsulfinyl, C₁-C₄-Alkylsulfonyl, C₁- C₃-Alkoxy-C₁-C₃-alkyl, C₃-C₆-Cycloalkyl; C₁-C₄-Halogenalkyl, C₁-C₄-Halogenalkylthio, C₁- C₄-Halogenalkylsulfinyl, C₁-C₄-Halogenalkylsulfonyl, Halogen-C₁-C₃-alkoxy-C₁-C₃-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)carbonyl-C₁-C₃- alkyl; Halogen-(C₁-C₃-alkyl)carbonyl-C₁-C₃-alkyl, Halogen-(C₁-C₃-alkoxy)carbonyl-C₁-C₃- alkyl mit jeweils 1 bis 13 Fluor-, Chlor- und/oder Bromatomen; (C₁-C₆-Alkyl)carbonyl, (C₁-C₄-Alkoxy)carbonyl, (C₁-C₄-Alkylthio)carbonyl, (C₁-C₃-Alkoxy- C₁-C₃-alkyl)carbonyl, (C₃-C₄-Alkenyloxy)carbonyl, (C₃-C₄-Alkinyloxy)carbonyl, (C₃-C₆- Cycloalkyl)carbonyl; (C₁-C₄-Halogenalkyl)carbonyl, (C₁-C₄-Halogenalkoxy)carbonyl, (C₁- C₄-Halogenalkylthio)carbonyl, (Halogen-C₁-C₃-alkoxy-C₁-C₃-alkyl)carbonyl, (C₃-C₄-Halo- genalkenyloxy)carbonyl, (C₃-C₄-Halogenalkinyloxy)carbonyl, (C₃-C₆-Halogencycloalkyl)- carbonyl mit jeweils 1 bis 9 Fluor-, Chlor- und/oder Bromatomen; oder -CH₂-C≡C-R^{1-A}, -CH₂-CH=CH-R^{1-A}, -CH=C=CH-R^{1-A}, -C(=O)C(=O)R², -CONR³R⁴ oder -CH₂NR⁵R⁶.
- R¹: steht besonders bevorzugt für Wasserstoff, Methyl, Ethyl, n- oder iso-Propyl, n-, iso-, sec- oder tert-Butyl, Pentyl oder Hexyl, Methylsulfinyl, Ethylsulfinyl, n- oder iso-Propylsulfinyl, n-, iso-, sec- oder tert-Butylsulfinyl, Methylsulfonyl, Ethylsulfonyl, n- oder iso-Propylsulfonyl, n-, iso-, sec- oder tert-Butylsulfonyl, Methoxymethyl, Methoxyethyl, Ethoxymethyl, Ethoxyethyl, Cyclo- propyl, Cyclopentyl, Cyclohexyl, Trifluormethyl, Trichlormethyl, Trifluorethyl, Difluormethyl- thio, Difluorchlormethylthio, Trifluormethylthio, Trifluormethylsulfinyl, Trifluormethylsulfonyl, Trifluormethoxymethyl; Formyl, -CH₂-CHO, -(CH₂)₂-CHO, -CH₂-CO-CH₃, -CH₂-CO-CH₂CH₃, -CH₂-CO-CH(CH₃)₂, -(CH₂)₂-CO-CH₃, -(CH₂)₂-CO-CH₂CH₃, -(CH₂)₂-CO-CH(CH₃)₂, -CH₂-CO₂CH₃, -CH₂-CO₂CH₂CH₃, -CH₂-CO₂CH(CH₃)₂, -(CH₂)₂-CO₂CH₃, -(CH₂)₂-CO₂CH₂CH₃, -(CH₂)₂-CO₂CH(CH₃)₂, -CH₂-CO-CF₃, -CH₂-CO-CCl₃, -CH₂-CO-CH₂CF₃, -CH₂-CO-CH₂CCl₃, -(CH₂)₂-CO-CH₂CF₃, -(CH₂)₂-CO-CH₂CCl₃, -CH₂-CO₂CH₂CF₃, -CH₂-CO₂CF₂CF₃, -CH₂-CO₂CH₂CCl₃, -CH₂-CO₂CCl₂CCl₃, -(CH₂)₂-CO₂CH₂CF₃, -(CH₂)₂-CO₂CF₂CF₃, -(CH₂)₂-CO₂CH₂CCl₃, -(CH₂)₂-CO₂CCl₂CCl₃; Methylcarbonyl, Ethylcarbonyl, n-Propylcarbonyl, iso-Propylcarbonyl, tert-Butylcarbonyl, Methoxycarbonyl, Ethoxycarbonyl, iso-Propoxycarbonyl, tert-Butoxycarbonyl, Methylthio- carbonyl, Ethylthiocarbonyl, iso-Propylthiocarbonyl, tert-Butylthiocarbonyl, Methoxymethylcarbonyl, Ethoxymethylcarbonyl, Cyclopropylcarbonyl; Trifluormethylcar- bonyl, Trifluormethoxycarbonyl, Trifluormethylthiocarbonyl, oder -CH₂-C≡C-R^{1-A}, -CH₂-CH=CH-R^{1-A}, -CH=C=CH-R^{1-A}, -C(=O)C(=O)R², -CONR³R⁴ oder -CH₂NR⁵R⁶.
- R¹: steht ganz besonders bevorzugt für Wasserstoff, Methyl, Methoxymethyl, Methoxymethyl- carbonyl, Ethoxymethylcarbonyl, Formyl, -CH₂-C≡CH, -CH₂-CH=CH₂, -CH=C=CH₂, -CH₂-CHO, -(CH₂)₂-CHO, -CH₂-CO-CH₃, -CH₂-CO-CH₂CH₃, -CH₂-CO-CH(CH₃)₂, -C(=O)CHO, -C(=O)C(=O)CH₃, -C(=O)C(=O)CH₂OCH₃, -C(=O)CO₂CH₃, -C(=O)CO₂CH₂CH₃.
- R^{1-A}: steht bevorzugt für Wasserstoff, C₁-C₄-Alkyl, C₁-C₄-Halogenalkyl, C₂-C₄-Alkenyl C₂-C₄- Alkinyl, C₃-C₆-Cycloalkyl, (C₁-C₄-Alkoxy)carbonyl, oder Cyano.
- R^{1-A}: steht besonders bevorzugt für Wasserstoff, Methyl oder Ethyl.
- R²: steht bevorzugt für Wasserstoff, C₁-C₆-Alkyl, C₁-C₄-Alkoxy, C₁-C₃-Alkoxy-C₁-C₃-alkyl, C₃- C₆-Cycloalkyl; C₁-C₄-Halogenalkyl, C₁-C₄-Halogenalkoxy, Halogen-C₁-C₃-alkoxy-C₁-C₃- alkyl, C₃-C₆-Halogencycloalkyl mit jeweils 1 bis 9 Fluor-, Chlor- und/oder Bromatomen.
- R²: steht besonders bevorzugt für Wasserstoff, Methyl, Ethyl, n- oder iso-Propyl, tert-Butyl, Methoxy, Ethoxy, n- oder iso-Propoxy, tert-Butoxy, Methoxymethyl, Cyclopropyl; Trifluor- methyl, Trifluormethoxy.
- R³ und R⁴: stehen unabhängig voneinander bevorzugt für Wasserstoff, C₁-C₆-Alkyl, C₁-C₃-Alkoxy- C₁-C₃-alkyl, C₃-C₆-Cycloakyl; C₁-C₄-Halogenalkyl, Halogen-C₁-C₃-alkoxy-C₁-C₃-alkyl, C₃- C₆-Halogencycloalkyl mit jeweils 1 bis 9 Fluor-, Chlor- und/oder Bromatomen.
- R³ und R⁴: bilden außerdem gemeinsam mit dem Stickstoffatom, an das sie gebunden sind, bevorzugt einen gegebenenfalls einfach bis vierfach, gleich oder verschieden durch Halogen oder C₁-C₄-Alkyl substituierten gesättigten Heterocyclus mit 5 oder 6 Ringatomen, wobei der Heterocyclus 1 oder 2 weitere, nicht benachbarte Heteroatome aus der Reihe Sauerstoff, Schwefel oder NR⁷ enthalten kann.
- R³ und R⁴: stehen unabhängig voneinander besonders bevorzugt für Wasserstoff, Methyl, Ethyl, n- oder iso-Propyl, n-, iso-, sec- oder tert-Butyl, Methoxymethyl, Methoxyethyl, Ethoxymethyl, Ethoxyethyl, Cyclopropyl, Cyclopentyl, Cyclohexyl; Trifluormethyl, Trichlormethyl, Trifluorethyl, Trifluormethoxymethyl.
- R³ und R⁴: bilden außerdem gemeinsam mit dem Stickstoffatom, an das sie gebunden sind, besonders bevorzugt einen gegebenenfalls einfach bis vierfach, gleich oder verschieden durch Fluor, Chlor, Brom oder Methyl substituierten gesättigten Heterocyclus aus der Reihe Morpholin, Thiomorpholin oder Piperazin, wobei das Piperazin am zweiten Stickstoffatom durch R⁷ substituiert sein kann.
- R⁵ und R⁶: stehen unabhängig voneinander bevorzugt für Wasserstoff, C₁-C₆-Alkyl, C₃-C₆-Cycloal- kyl; C₁-C₄-Halogenalkyl, C₃-C₆-Halogencycloalkyl mit jeweils 1 bis 9 Fluor-, Chlor- und/oder Bromatomen.
- R⁵ und R⁶: bilden außerdem gemeinsam mit dem Stickstoffatom, an das sie gebunden sind, bevorzugt einen gegebenenfalls einfach oder mehrfach, gleich oder verschieden durch Halogen oder C₁- C₄-Alkyl substituierten gesättigten Heterocyclus mit 5 oder 6 Ringatomen, wobei der Heterocyclus 1 oder 2 weitere, nicht benachbarte Heteroatome aus der Reihe Sauerstoff, Schwefel oder NR⁷ enthalten kann.
- R⁵ und R⁶: stehen unabhängig voneinander besonders bevorzugt für Wasserstoff, Methyl, Ethyl, n- oder iso-Propyl, n-, iso-, sec- oder tert-Butyl, Methoxymethyl, Methoxyethyl, Ethoxyethyl, Ethoxyethyl, Cyclopropyl, Cyclopentyl, Cyclohexyl; Trifluormethyl, Trichlormethyl, Trifluorethyl, Trifluormethoxymethyl.
- R⁵ und R⁶: bilden außerdem gemeinsam mit dem Stickstoffatom, an das sie gebunden sind, besonders bevorzugt einen gegebenenfalls einfach bis vierfach, gleich oder verschieden durch Fluor, Chlor, Brom oder Methyl substituierten gesättigten Heterocyclus aus der Reihe Morpholin, Thiomorpholin oder Piperazin, wobei das Piperazin am zweiten Stickstoffatom durch R⁷ substituiert sein kann.
- R⁷: steht bevorzugt für Wasserstoff oder C₁-C₄-Alkyl.
- R⁷: steht besonders bevorzugt für Wasserstoff, Methyl, Ethyl, n- oder iso-Propyl, n-, iso-, sec- oder tert-Butyl.

Bevorzugt stehen X¹, X² und X³ jeweils für CR⁸ und X⁴ für N.
Bevorzugt stehen außerdem X¹, X² und X⁴ jeweils für CR⁸ und X³ für N.
Bevorzugt stehen außerdem X¹, X³ und X⁴ jeweils für CR⁸ und X² für N.
Bevorzugt stehen außerdem X², X³ und X⁴ jeweils für CR⁸ und X¹ für N.
Bevorzugt stehen X² und X⁴ jeweils für CR⁸ und X¹ und X³ jeweils für N.
Bevorzugt stehen außerdem X² und X³ jeweils für CR⁸ und X¹ und X⁴ jeweils für N.
Bevorzugt stehen außerdem X³ und X⁴ jeweils für CR⁸ und X¹ und X² jeweils für N.
Bevorzugt stehen außerdem X¹ und X⁴ jeweils für CR⁸ und X² und X³ jeweils für N.
Besonders bevorzugt stehen X² und X⁴ jeweils für CR⁸ und X¹ und X³ jeweils für N.
Ganz besonders bevorzugt stehen X² und X⁴ jeweils für CH und X¹ und X³ jeweils für N.
- R⁸: steht bevorzugt für Wasserstoff, Fluor, Chlor, Methyl oder Trifluormethyl.
- R⁸: steht besonders bevorzugt für Wasserstoff.
- R⁸: steht außerdem besonders bevorzugt für Fluor.
- R⁸: steht außerdem besonders bevorzugt für Chlor.
- R⁸: steht außerdem besonders bevorzugt für Methyl.
- R⁸: steht außerdem besonders bevorzugt für Trifluormethyl.
- A: steht bevorzugt für einen der Reste A1, A2, A3, A4, A5, A6, A9, A10, A11, A12 oder A17.
- A: steht besonders bevorzugt für einen der Reste A1, A2, A4, A5, A6, A9, A11, A16, A17.
- A: steht ganz besonders bevorzugt für den Rest A1.
- A: steht außerdem ganz besonders bevorzugt für den Rest A2.
- A: steht außerdem ganz besonders bevorzugt für den Rest A4.
- A: steht außerdem ganz besonders bevorzugt für den Rest A5.
- A: steht außerdem ganz besonders bevorzugt für den Rest A6.
- A: steht außerdem ganz besonders bevorzugt für den Rest A9.
- A: steht außerdem ganz besonders bevorzugt für den Rest A11.
- A: steht außerdem ganz besonders bevorzugt für den Rest A16.
- A: steht außerdem ganz besonders bevorzugt für den Rest A17.
- R⁹: steht bevorzugt für Wasserstoff, Cyano, Fluor, Chlor, Brom, Iod, Methyl, Ethyl, iso-Propyl, Methoxy, Ethoxy, Methylthio, Ethylthio, Cyclopropyl, C₁-C₂-Halogenalkyl, C₁-C₂-Halo- genalkoxy mit jeweils 1 bis 5 Fluor, Chlor und/oder Bromatomen, Trifluormethylthio, Di- fluormethylthio, Aminocarbonyl, Aminocarbonylmethyl oder Aminocarbonylethyl.
- R⁹: steht besonders bevorzugt für Wasserstoff, Fluor, Chlor, Brom, Iod, Methyl, Ethyl, iso- Propyl, Monofluormethyl, Monofluorethyl, Difluormethyl, Trifluormethyl, Difluorchlor- methyl, Trichlormethyl, Dichlormethyl, Cyclopropyl, Methoxy, Ethoxy, Trifluormethoxy, Trichlormethoxy, Methylthio, Ethylthio, Trifluormethylthio oder Difluormethylthio.
- R⁹: steht ganz besonders bevorzugt für Wasserstoff, Fluor, Chlor, Brom, lod, Methyl, iso- Propyl, Monofluormethyl, Monofluorethyl, Difluormethyl, Trifluormethyl, Difluor- chlormethyl oder Trichlormethyl.
- R⁹: steht insbesondere bevorzugt für Methyl, Difluormethyl, Trifluormethyl oder 1-Fluorethyl.
- R¹⁰: steht bevorzugt für Wasserstoff, Fluor, Chlor, Brom, Iod, Methyl, Ethyl, Methoxy, Ethoxy, Methylthio oder Ethylthio.
- R¹⁰: steht besonders bevorzugt für Wasserstoff, Fluor, Chlor, Brom, Iod oder Methyl.
- R¹⁰: steht ganz besonders bevorzugt für Wasserstoff, Fluor, Chlor oder Methyl.
- R¹¹: steht bevorzugt für Wasserstoff, Methyl, Ethyl, n-Propyl, iso-Propyl, C₁-C₂-Halogenalkyl mit 1 bis 5 Fluor, Chlor und/oder Bromatomen, Hydroxymethyl, Hydroxyethyl, Cyclo- propyl, Cyclopentyl, Cyclohexyl, Phenyl, Methylcarbonyl, n-Propylcarbonyl, tert-Butyl- carbonyl, Methoxycarbonyl, iso-Propoxycarbonyl, tert-Butoxycarbonyl, Methoxymethyl- carbonyl.
- R¹¹: steht besonders bevorzugt für Wasserstoff, Methyl, Ethyl, iso-Propyl, Trifluormethyl, Di- fluormethyl, Hydroxymethyl, Hydroxyethyl, Phenyl, Methylcarbonyl, iso-Propoxycarbonyl, tert-Butoxycarbonyl, Methoxymethylcarbonyl.
- R¹¹: steht ganz besonders bevorzugt für Wasserstoff, Methyl, Trifluormethyl, Phenyl oder Methylcarbonyl.
- R¹¹: steht insbesondere bevorzugt für Methyl.
- R¹² und R¹³: stehen unabhängig voneinander bevorzugt für Wasserstoff, Fluor, Chlor, Brom, Methyl, Ethyl oder C₁-C₂-Halogenalkyl mit 1 bis 5 Fluor, Chlor und/oder Bromatomen.
- R¹² und R¹³: stehen unabhängig voneinander besonders bevorzugt für Wasserstoff Fluor, Chlor, Brom, Methyl, Ethyl, Difluormethyl, Trifluormethyl, Difluorchlormethyl oder Trichlormethyl.
- R¹² und R¹³: stehen unabhängig voneinander ganz besonders bevorzugt für Wasserstoff, Fluor, Chlor, Brom, Methyl, Ethyl, Difluormethyl, Trifluormethyl oder Trichlormethyl.
- R¹² und R¹³: stehen insbesondere bevorzugt jeweils für Wasserstoff.
- R¹⁴: steht bevorzugt für Fluor, Chlor, Brom, Cyano, Methyl, Ethyl, C₁-C₂-Halogenalkyl oder C₁- C₂-Halogenalkoxy mit jeweils 1 bis 5 Fluor, Chlor und/oder Bromatomen.
- R¹⁴: steht besonders bevorzugt für Fluor, Chlor, Brom, Cyano, Methyl, Trifluormethyl, Trifluormethoxy, Difluormethoxy, Difluorchlormethoxy oder Trichlormethoxy.
- R¹⁴: steht ganz besonders bevorzugt für Fluor, Chlor, Brom, Iod, Methyl, Trifluormethyl oder Trifluormethoxy.
- R¹⁴: steht insbesondere bevorzugt für Methyl oder Trifluormethyl.
- R¹⁵ und R¹⁶: stehen unabhängig voneinander bevorzugt für Wasserstoff, Fluor, Chlor, Brom, Methyl, Ethyl oder C₁-C₂-Halogenalkyl mit 1 bis 5 Fluor, Chlor und/oder Bromatomen.
- R¹⁵ und R¹⁶: stehen unabhängig voneinander besonders bevorzugt für Wasserstoff, Fluor, Chlor, Brom, Methyl, Ethyl, Difluormethyl, Trifluormethyl, Difluorchlormethyl oder Trichlormethyl.
- R¹⁵ und R¹⁶: stehen unabhängig voneinander ganz besonders bevorzugt für Wasserstoff, Fluor, Chlor, Brom, Methyl, Ethyl, Difluormethyl, Trifluormethyl oder Trichlormethyl.
- R¹⁵ und R¹⁶: stehen insbesondere bevorzugt jeweils für Wasserstoff.
- R¹⁷: steht bevorzugt für Wasserstoff, Methyl, Ethyl oder C₁-C₂-Halogenalkyl mit 1 bis 5 Fluor, Chlor und/oder Bromatomen.
- R¹⁷: steht besonders bevorzugt für Wasserstoff, Methyl oder Trifluormethyl.
- R¹⁷: steht ganz besonders bevorzugt für Methyl oder Trifluormethyl.
- R¹⁸: steht bevorzugt für Wasserstoff, Fluor, Chlor, Brom, Iod, Hydroxy, Cyano, C₁-C₄-Alkyl, C₁-C₂-Halogenalkyl, C₁-C₂-Halogenalkoxy oder C₁-C₂-Halogenalkylthio mit jeweils 1 bis 5 Fluor, Chlor und/oder Bromatomen.
- R¹⁸: steht besonders bevorzugt für Wasserstoff, Fluor, Chlor, Brom, Iod, Hydroxy, Cyano, Methyl, Ethyl, n-Propyl, iso-Propyl, n-Butyl, iso-Butyl, sec-Butyl, tert-Butyl, Difluormethyl, Trifluor- methyl, Difluorchlormethyl, Trichlormethyl, Trifluormethoxy, Difluormethoxy, Difluorchlor- methoxy, Trichlormethoxy, Trifluormethylthio, Difluormethylthio, Difluorchlormethylthio oder Trichlormethylthio.
- R¹⁸: steht ganz besonders bevorzugt für Wasserstoff, Fluor, Chlor, Brom, Iod, Methyl, Difluor- methyl, Trifluormethyl oder Trichlormethyl.
- R¹⁸: steht insbesondere bevorzugt für Iod, Methyl, Difluormethyl oder Trifluormethyl.
- R¹⁹: steht bevorzugt für Fluor, Chlor, Brom, Iod, Hydroxy, Cyano, C₁-C₄-Alkyl, Methoxy, Ethoxy, Methylthio, Ethylthio, Difluormethylthio, Trifluormethylthio, C₁-C₂-Halogenalkyl oder C₁-C₂-Halogenalkoxy mit jeweils 1 bis 5 Fluor, Chlor und/oder Bromatomen.
- R¹⁹: steht besonders bevorzugt für Fluor, Chlor, Brom, Iod, Hydroxy, Cyano, Methyl, Ethyl, n- Propyl, iso-Propyl, n-Butyl, iso-Butyl, sec-Butyl, tert-Butyl, Trifluormethyl, Difluormethyl, Difluorchlormethyl, Trichlormethyl, Methoxy, Ethoxy, Methylthio, Ethylthio, Difluorme- thylthio, Trifluormethylthio, Trifluormethoxy, Difluormethoxy, Difluorchlormethoxy oder Trichlormethoxy.
- R¹⁹: steht ganz besonders bevorzugt für Fluor, Chlor, Brom, Iod, Methyl, Trifluormethyl, Di- fluormethyl oder Trichlormethyl.
- R²⁰: steht bevorzugt für Wasserstoff, Fluor, Chlor, Brom, Iod, Cyano, C₁-C₄-Alkyl, Methoxy, Ethoxy, Methylthio, Ethylthio, C₁-C₂-Halogenalkyl oder C₁-C₂-Halogenalkoxy mit jeweils 1 bis 5 Fluor, Chlor und/oder Bromatomen, C₁-C₂-Alkylsulphinyl oder C₁-C₂-Alkylsulphonyl.
- R²⁰: steht besonders bevorzugt für Wasserstoff, Fluor, Chlor, Brom, Iod, Cyano, n-Propyl, iso- Propyl, n-Butyl, iso-Butyl, sec-Butyl, tert-Butyl, Trifluormethyl, Difluormethyl, Difluor- chlormethyl, Trichlormethyl, Methoxy, Ethoxy, Methylthio, Ethylthio, Trifluormethoxy, Difluormethoxy, Difluorchlormethoxy, Trichlormethoxy, Methylsulphinyl oder Methyl- sulphonyl.
- R²⁰: steht ganz besonders bevorzugt für Wasserstoff, Fluor, Chlor, Brom, Iod, n-Propyl, iso- Propyl, n-Butyl, iso-Butyl, sec-Butyl, tert-Butyl, Trifluormethyl, Difluormethyl, Tri- chlormethyl, Methylsulphinyl oder Methylsulphonyl.
- R²⁰: steht insbesondere bevorzugt für Wasserstoff.
- R²¹: steht bevorzugt für Methyl, Ethyl oder C₁-C₂-Halogenalkyl mit 1 bis 5 Fluor, Chlor und/oder Bromatomen.
- R²¹: steht besonders bevorzugt für Methyl, Ethyl, Trifluormethyl, Difluormethyl, Difluor- chlormethyl oder Trichlormethyl.
- R²²: steht bevorzugt für Methyl oder Ethyl.
- R²²: steht besonders bevorzugt für Methyl.
- Q¹: steht bevorzugt für S (Schwefel), SO₂ oder CH₂.
- Q¹: steht besonders bevorzugt für S (Schwefel) oder CH₂.
- Q¹: steht ganz besonders bevorzugt für S (Schwefel).
- p: steht bevorzugt für 0 oder 1.
- p: steht besonders bevorzugt für 0.
- R²³: steht bevorzugt für Methyl, Ethyl oder C₁-C₂-Halogenalkyl mit 1 bis 5 Fluor, Chlor und/oder Bromatomen.
- R²³: steht besonders bevorzugt für Methyl, Ethyl, Trifluormethyl, Difluormethyl, Difluorchlor- methyl oder Trichlormethyl.
- R²³: steht ganz besonders bevorzugt für Methyl, Trifluormethyl, Difluormethyl oder Trichlor- methyl.
- R²⁴: steht bevorzugt für Methyl, Ethyl oder C₁-C₂-Halogenalkyl mit 1 bis 5 Fluor, Chlor und/oder Bromatomen.
- R²⁴: steht besonders bevorzugt für Methyl, Ethyl, Trifluormethyl, Difluormethyl, Difluorchlor- methyl oder Trichlormethyl.
- R²⁴: steht ganz besonders bevorzugt für Methyl, Trifluormethyl, Difluormethyl oder Trichlormethyl.
- R²⁵ und R²⁶: stehen unabhängig voneinander bevorzugt für Wasserstoff, Fluor, Chlor, Brom, Amino, Methyl, Ethyl oder C₁-C₂-Halogenalkyl mit 1 bis 5 Fluor, Chlor und/oder Bromatomen.
- R²⁵ und R²⁶: stehen unabhängig voneinander besonders bevorzugt für Wasserstoff, Fluor, Chlor, Brom, Methyl, Ethyl, Trifluormethyl, Difluormethyl, Difluorchlormethyl oder Trichlormethyl.
- R²⁵ und R²⁶: stehen unabhängig voneinander ganz besonders bevorzugt für Wasserstoff, Fluor, Chlor, Brom, Methyl, Trifluormethyl, Difluormethyl oder Trichlormethyl.
- R²⁵ und R²⁶: stehen insbesondere bevorzugt jeweils für Wasserstoff.
- R²⁷: steht bevorzugt für Wasserstoff, Methyl, Ethyl oder C₁-C₂-Halogenalkyl mit 1 bis 5 Fluor, Chlor und/oder Bromatomen.
- R²⁷: steht besonders bevorzugt für Wasserstoff, Methyl, Ethyl, Trifluormethyl, Difluormethyl, Difluorchlormethyl oder Trichlormethyl.
- R²⁷: steht ganz besonders bevorzugt für Wasserstoff, Methyl, Trifluormethyl, Difluormethyl oder Trichlormethyl.
- R²⁷: steht insbesondere bevorzugt für Methyl.
- R²⁸ und R²⁹: stehen unabhängig voneinander bevorzugt für Wasserstoff, Fluor, Chlor, Brom, Amino, Nitro, Methyl, Ethyl oder C₁-C₂-Halogenalkyl mit 1 bis 5 Fluor, Chlor und/oder Bromatomen.
- R²⁸ und R²⁹: stehen unabhängig voneinander besonders bevorzugt für Wasserstoff, Fluor, Chlor, Brom, Nitro, Methyl, Ethyl, Trifluormethyl, Difluormethyl, Difluorchlormethyl oder Trichlormethyl.
- R²⁸ und R²⁹: stehen unabhängig voneinander ganz besonders bevorzugt für Wasserstoff, Fluor, Chlor, Brom, Methyl, Trifluormethyl, Difluormethyl oder Trichlormethyl.
- R²⁸ und R²⁹: stehen insbesondere bevorzugt jeweils für Wasserstoff.
- R³⁰: steht bevorzugt für Wasserstoff, Fluor, Chlor, Brom, Methyl, Ethyl oder C₁-C₂- Halogenalkyl mit 1 bis 5 Fluor, Chlor und/oder Bromatomen,
- R³⁰: steht besonders bevorzugt für Wasserstoff, Fluor, Chlor, Brom, Methyl, Ethyl, Trifluorme- thyl, Difluormethyl, Difluorchlormethyl oder Trichlormethyl.
- R³⁰: steht ganz besonders bevorzugt für Wasserstoff, Fluor, Chlor, Brom, Methyl, Trifluor- methyl, Difluormethyl oder Trichlormethyl.
- R³⁰: steht insbesondere bevorzugt für Methyl.
- R³¹: steht bevorzugt für Wasserstoff, Fluor, Chlor, Brom, Amino, C₁-C₄-Alkylamino, Di(C₁-C₄- alkyl)amino, Cyano, Methyl, Ethyl oder C₁-C₂-Halogenalkyl mit 1 bis 5 Fluor, Chlor und/oder Bromatomen.
- R³¹: steht besonders bevorzugt für Wasserstoff, Fluor, Chlor, Brom, Amino, Methylamino, Dimethylamino, Cyano, Methyl, Ethyl, Trifluormethyl, Difluormethyl, Difluorchlormethyl oder Trichlormethyl.
- R³¹: steht ganz besonders bevorzugt für Wasserstoff, Fluor, Chlor, Brom, Amino, Dimethylamino, Methyl, Trifluormethyl, Difluormethyl oder Trichlormethyl.
- R³¹: steht insbesondere bevorzugt für Amino, Methylamino, Dimethylamino, Methyl oder Trifluormethyl.
- R³²: steht bevorzugt für Fluor, Chlor, Brom, Methyl, Ethyl oder C₁-C₂-Halogenalkyl mit 1 bis 5 Fluor, Chlor und/oder Bromatomen.
- R³²: steht besonders bevorzugt für Fluor, Chlor, Brom, Methyl, Ethyl, Trifluormethyl, Difluormethyl, Difluorchlormethyl oder Trichlormethyl.
- R³²: steht ganz besonders bevorzugt für Fluor, Chlor, Brom, Methyl, Trifluormethyl, Difluor- methyl oder Trichlormethyl.
- R³²: steht insbesondere bevorzugt für Methyl, Trifluormethyl oder Difluormethyl.
- R³³: steht bevorzugt für Wasserstoff, Fluor, Chlor, Brom, Amino, C₁-C₄-Alkylamino, Di(C₁-C₄- alkyl)amino, Cyano, Methyl, Ethyl oder C₁-C₂-Halogenalkyl mit 1 bis 5 Fluor, Chlor und/oder Bromatomen.
- R³³: steht besonders bevorzugt für Wasserstoff, Fluor, Chlor, Brom, Amino, Methylamino, Dimethylamino, Cyano, Methyl, Ethyl, Trifluormethyl, Difluormethyl, Difluorchlormethyl oder Trichlormethyl.
- R³³: steht ganz besonders bevorzugt für Wasserstoff, Fluor, Chlor, Brom, Amino, Methylamino, Dimethylamino, Methyl, Trifluormethyl, Difluormethyl oder Trichlormethyl.
- R³³: steht insbesondere bevorzugt für Amino, Methylamino, Dimethylamino, Methyl oder Trifluormethyl.
- R³⁴: steht bevorzugt für Fluor, Chlor, Brom, Methyl, Ethyl oder C₁-C₂-Halogenalkyl mit 1 bis 5 Fluor, Chlor und/oder Bromatomen.
- R³⁴: steht besonders bevorzugt für Fluor, Chlor, Brom, Methyl, Ethyl, Trifluormethyl, Difluormethyl, Difluorchlormethyl oder Trichlormethyl.
- R³⁴: steht ganz besonders bevorzugt für Fluor, Chlor, Brom, Methyl, Trifluormethyl, Difluormethyl oder Trichlormethyl.
- R³⁴: steht insbesondere bevorzugt für Methyl, Trifluormethyl oder Difluormethyl.
- R³⁵: steht bevorzugt für Fluor, Chlor, Brom, Methyl, Ethyl oder C₁-C₂-Halogenalkyl mit 1 bis 5 Fluor, Chlor und/oder Bromatomen.
- R³⁵: steht besonders bevorzugt für Fluor, Chlor, Brom, Methyl, Ethyl, Trifluormethyl, Difluormethyl, Difluorchlormethyl oder Trichlormethyl.
- R³⁵: steht ganz besonders bevorzugt für Fluor, Chlor, Brom, Methyl, Trifluormethyl, Difluormethyl oder Trichlormethyl.
- R³⁶: steht bevorzugt für Wasserstoff, Methyl oder Ethyl.
- R³⁶: steht besonders bevorzugt für Methyl.
- R³⁷: steht bevorzugt für Fluor, Chlor, Brom, Methyl oder Ethyl.
- R³⁷: steht besonders bevorzugt für Fluor, Chlor oder Methyl.
- R³⁸: steht bevorzugt für Methyl, Ethyl oder C₁-C₂-Halogenalkyl mit 1 bis 5 Fluor, Chlor und/oder Bromatomen.
- R³⁸: steht besonders bevorzugt für Methyl, Ethyl, Trifluormethyl, Difluormethyl, Difluorchlor- methyl oder Trichlormethyl.
- R³⁸: steht besonders bevorzugt für Methyl, Trifluormethyl, Difluormethyl oder Trichlormethyl.
- R³⁸: steht insbesondere bevorzugt für Methyl oder Trifluormethyl.
- R³⁹: steht bevorzugt für Wasserstoff, Fluor, Chlor, Brom, Methyl, Ethyl oder C₁-C₂-Halogen- alkyl mit 1 bis 5 Fluor, Chlor und/oder Bromatomen.
- R³⁹: steht besonders bevorzugt für Wasserstoff, Fluor, Chlor, Brom, Methyl oder Trifluormethyl.
- R⁴⁰: steht bevorzugt für Fluor, Chlor, Brom, Iod, Hydroxy, C₁-C₄-Alkyl, Methoxy, Ethoxy, Methylthio, Ethylthio, Difluormethylthio, Trifluormethylthio, C₁-C₂-Halogenalkyloder C₁- C₂-Halogenalkoxy mit jeweils 1 bis 5 Fluor, Chlor und/oder Bromatomen.
- R⁴⁰: steht besonders bevorzugt für Fluor, Chlor, Brom, Iod, Methyl, Ethyl, n-Propyl, iso-Propyl, n-Butyl, iso-Butyl, sec-Butyl, tert-Butyl, Trifluormethyl, Difluormethyl, Difluorchlormethyl oder Trichlormethyl.
- R⁴⁰: steht ganz besonders bevorzugt für Fluor, Chlor, Brom, lod, Methyl, Trifluormethyl, Difluormethyl oder Trichlormethyl.
- R⁴¹: steht bevorzugt für Methyl, Ethyl, n-Propyl oder iso-Propyl.
- R⁴¹: steht besonders bevorzugt Methyl oder Ethyl.

Bevorzugt sind solche Verbindungen der Formel (I), in welcher alle Reste jeweils die oben genannten bevorzugten Bedeutungen haben.
Besonders bevorzugt sind solche Verbindungen der Formel (I), in welcher alle Reste jeweils die oben genannten besonders bevorzugten Bedeutungen haben.

Bevorzugt sind außerdem Verbindungen der Formel (I-b) 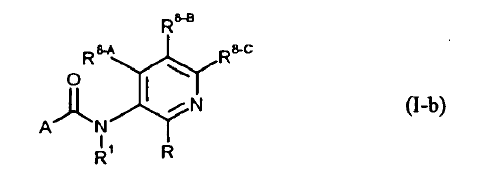 in welcher
R, R' und A die oben angegebenen Bedeutungen haben,
R^{8-A}, R^{8-B} und R^{8-C} jeweils unabhängig voneinander die Bedeutungen von R⁸ haben.

Besonders bevorzugt sind Verbindungen der Formel (I-b), in welcher die Reste R^{8-A}, R^{8-B} und R^{8-C} gleichzeitig für Wasserstoff stehen.
Außerdem besonders bevorzugt sind Verbindungen der Formel (I-b), in welcher die Reste R^{8-B} und R^{8-C} gleichzeitig für Wasserstoff stehen und der Rest R^{8-A} für Fluor oder Chlor steht.
Außerdem besonders bevorzugt sind Verbindungen der Formel (I-b), in welcher die Reste R^{8-A} und R^{8-B} gleichzeitig für Wasserstoff stehen und der Rest R^{8-C} für Methyl steht.

Bevorzugt sind außerdem Verbindungen der Formel (I-c) 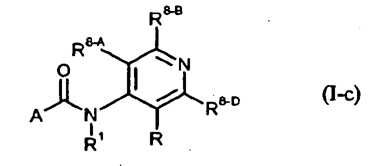 in welcher
R, R¹ und A die oben angegebenen Bedeutungen haben,
R^{8-A}, R^{8-B} und R^{8-D} jeweils unabhängig voneinander die Bedeutungen von R⁸ haben.

Besonders bevorzugt sind Verbindungen der Formel (I-c), in welcher die Reste R^{8-A}, R^{8-B} und R^{8-D} gleichzeitig für Wasserstoff stehen.
Außerdem besonders bevorzugt sind Verbindungen der Formel (I-c), in welcher die Reste R^{8-B} und R^{8-D} gleichzeitig für Wasserstoff stehen und der Rest R^{8-A} für Fluor oder Chlor steht.
Außerdem besonders bevorzugt sind Verbindungen der Formel (I-c), in welcher die Reste R^{8-A} und R^{8-B} gleichzeitig für Wasserstoff stehen und der Rest R^{8-D} für Methyl steht.

Bevorzugt sind außerdem Verbindungen der Formel (I-d) 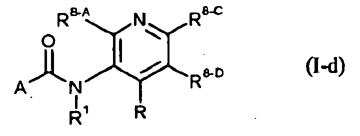 in welcher
R, R¹ und A die oben angegebenen Bedeutungen haben,
R^{8-A}, R^{8-C} und R^{8-D} jeweils unabhängig voneinander die Bedeutungen von R⁸ haben.

Besonders bevorzugt sind Verbindungen der Formel (I-d), in welcher die Reste R^{8-A}, R^{8-C} und R^{8-D} gleichzeitig für Wasserstoff stehen.
Außerdem besonders bevorzugt sind Verbindungen der Formel (I-d), in welcher die Reste R^{8-A} und R^{8-C} gleichzeitig für Wasserstoff stehen und der Rest R^{8-D} für Fluor, Chlor oder Methyl steht.

Bevorzugt sind außerdem Verbindungen der Formel (I-e) 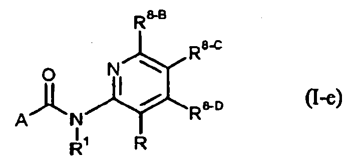 in welcher
R, R¹ und A die oben angegebenen Bedeutungen haben,
R^{8-B}, R^{8-C} und R^{8-D} jeweils unabhängig voneinander die Bedeutungen von R⁸ haben.

Besonders bevorzugt sind Verbindungen der Formel (I-e), in welcher die Reste R^{8-B}, R^{8-C} und R^{8-D} gleichzeitig für Wasserstoff stehen.
Außerdem besonders bevorzugt sind Verbindungen der Formel (I-e), in welcher die Reste R^{8-B} und R^{8-C} gleichzeitig für Wasserstoff stehen und der Rest R^{8-D} für Fluor, Chlor oder Methyl steht.

Bevorzugt sind außerdem Verbindungen der Formel (I-f) 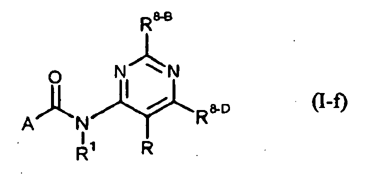 in welcher
R, R¹ und A die oben angegebenen Bedeutungen haben,
R^{8-B} und R^{8-D} jeweils unabhängig voneinander die Bedeutungen von R⁸ haben.

Besonders bevorzugt sind Verbindungen der Formel (I-f), in welcher die Reste R^{8-B} und R^{8-D} gleichzeitig für Wasserstoff stehen.
Außerdem besonders bevorzugt sind Verbindungen der Formel (I-f), in welcher der Rest R^{8-B} für Wasserstoff und der Rest R^{8-D} für Methyl oder Trifluormethyl steht.

Bevorzugt sind außerdem Verbindungen der Formel (I-g) 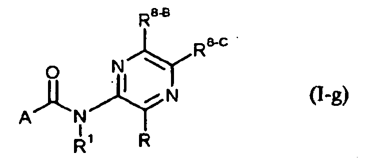 in welcher
R, R¹ und A die oben angegebenen Bedeutungen haben,
R^{8-B} und R^{8-C} jeweils unabhängig voneinander die Bedeutungen von R⁸ haben.

Besonders bevorzugt sind Verbindungen der Formel (I-g), in welcher die Reste R^{8-B} und R^{8-C} gleichzeitig für Wasserstoff stehen.
Außerdem besonders bevorzugt sind Verbindungen der Formel (I-g), in welcher der Rest R^{8-B} für Wasserstoff und der Rest R^{8-C} für Methyl oder Trifluormethyl steht.

Bevorzugt sind außerdem Verbindungen der Formel (I-h) 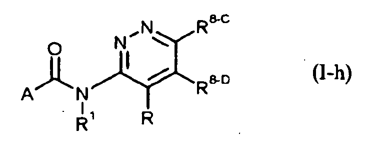 in welcher
R, R¹ und A die oben angegebenen Bedeutungen haben,
R^{8-C} und R^{8-D} jeweils unabhängig voneinander die Bedeutungen von R⁸ haben.

Besonders bevorzugt sind Verbindungen der Formel (I-h), in welcher die Reste R^{8-C} und R^{8-D} gleichzeitig für Wasserstoff stehen.
Außerdem besonders bevorzugt sind Verbindungen der Formel (I-h), in welcher der Rest R^{8-C} für Wasserstoff und der Rest R^{8-D} für Methyl oder Trifluormethyl steht.

Bevorzugt sind außerdem Verbindungen der Formel (I-j) 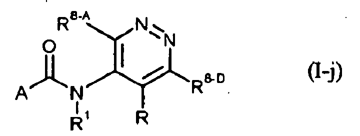 in welcher
R, R' und A die oben angegebenen Bedeutungen haben,
R^{8-A} und R^{8-D} jeweils unabhängig voneinander die Bedeutungen von R⁸ haben.

Besonders bevorzugt sind Verbindungen der Formel (I-j), in welcher die Reste R^{8-A} und R^{8-D} gleichzeitig für Wasserstoff stehen.
Außerdem besonders bevorzugt sind Verbindungen der Formel (I-j), in welcher der Rest R^{8-A} für Wasserstoff und der Rest R^{8-D} für Methyl oder Trifluormethyl steht.

Bevorzugt sind ebenfalls Verbindungen der Formel (I), (I-b), (1-c), (I-d), (I-e), (I-f), (I-g), (I-h), (I-j), in welchen R¹ für Wasserstoff steht.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend.

Verwendet man beispielsweise 2-(Trifluormethyl)benzoylchlorid und 5-(4-Chlorphenyl)-4-pyrimidinylamin als Ausgangsstoffe sowie ein Base, so kann der Verlauf des erfindungsgemäßen Verfahrens (a) durch folgende Reaktionsgleichung veranschaulicht werden:

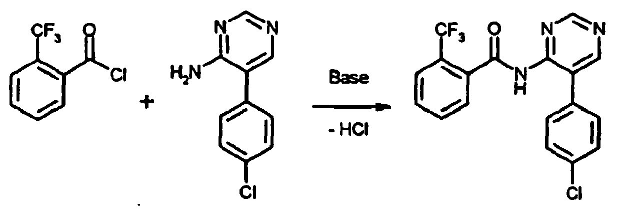

Die zur Durchführung des erfindungsgemäßen Verfahrens (a) als Ausgangsstoffe benötigten Carbonsäurehalogenide sind durch die Formel (II) allgemein definiert. In dieser Formel (II) steht A bevorzugt, besonders bevorzugt, ganz besonders bevorzugt bzw. insbesondere bevorzugt für diejenigen Bedeutungen, die bereits in Zusammenhang mit der Beschreibung der erfindungsgemäßen. Stoffe der Formel (I) als bevorzugt, besonders bevorzugt usw. für diesen Rest angegeben wurden. X⁵ steht bevorzugt für Fluor, Chlor oder Hydroxy, besonders bevorzugt für Chlor oder Hydroxy.

Die Carbonsäurehalogenide der Formel (II) sind bekannt und/oder lassen sich nach bekannten Verfahren herstellen (vgl. z.B. EP-A 0 545 099, JP-A 01-290662 und US 5,093,347).

Die weiterhin zur Durchführung des erfindungsgemäßen Verfahrens (a) als Ausgangsstoffe benötigten Anilin-Derivate sind durch die Formel (III) allgemein definiert. In dieser Formel (III) stehen R, R¹, X¹, X², X³ und X⁴ bevorzugt, besonders bevorzugt, ganz besonders bevorzugt bzw. insbesondere bevorzugt für diejenigen Bedeutungen, die bereits in Zusammenhang mit der Beschreibung der erfindungsgemäßen Stoffe der Formel (I) als bevorzugt, besonders bevorzugt usw. für diese Reste angegeben wurden.

Die Anilin-Derivate der Formel (III) sind teilweise bekannt und/oder lassen sich nach bekannten Verfahren herstellen (vgl. z.B. J. Org. Chem 1972, 21, 3216-3220).

Neu, und damit ebenfalls Gegenstand dieser Anmeldung, sind Verbindungen der Formel (III-a) 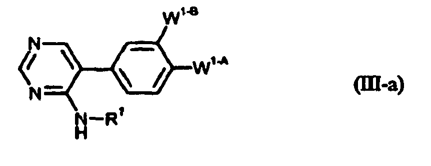 in welcher
R¹ die oben angegebenen Bedeutungen hat,
und
W^{1-A} und W^{1-B} entweder gleichzeitig für Chlor stehen
oder
W^{1-A} für Trifluormethyl oder Trifluormethoxy steht und W^{1-B} für Wasserstoff steht.

Verbindungen der Formel (III-a) werden erhalten, indem man
(c) Phenylacetonitrile der Formel (V) 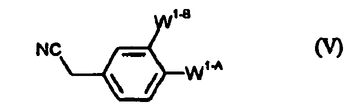 in welcher W^{1-A} und W^{1-B} die oben angegebenen Bedeutungen haben,
   mit Tris(formamino)methan und Formamid umsetzt
   und die so erhaltenen Anilinderivate der Formel (III-b). 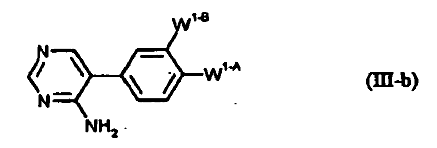 in welcher W^{1-A} und W^{1-B} die oben angegebenen Bedeutungen haben,
   gegebenenfalls mit Halogeniden der Formel (IV)

   R^{1-B}-Hal (IV)

   in welcher R^{1-B} und Hal die oben angegebenen Bedeutungen haben,
   in Gegenwart einer Base und in Gegenwart eines Verdünnungsmittels umsetzt [für diesen Schritt gelten die Reaktionsbedingungen des Verfahren (b) entsprechend].

Die zur Durchführung des erfindungsgemäßen Verfahrens (c) als Ausgangsstoffe benötigten Stoffe Phenylacetonitrile der Formel (V) sind bekannte Synthesechemikalien.

Verwendet man N-[5-(4-Chlorphenyl)pyrimidin4-yl]-2-(trifluormethyl)benzamid und 2-(Trifluormethyl)benzoylchlorid als Ausgangsstoffe, so kann der Verlauf des erfindungsgemäßen Verfahrens (b) durch das folgende Formelschema veranschaulicht werden:

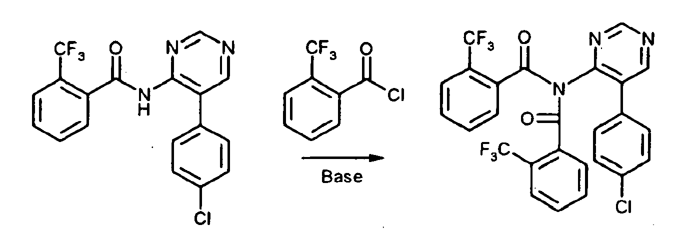

Die zur Durchführung des erfindungsgemäßen Verfahrens (b) als Ausgangsstoffe benötigten Carboxamide sind durch die Formel (I-a) allgemein definiert. In dieser Formel (I-a) haben R, X¹, X², X³, X⁴ und A bevorzugt, besonders bevorzugt, ganz besonders bevorzugt bzw. insbesondere bevorzugt diejenigen Bedeutungen, die bereits im Zusammenhang mit der Beschreibung der erfindungsgemäßen Verbindungen der Formel (I) als bevorzugt, besonders bevorzugt usw. für diese Reste angegeben wurden.

Die Verbindungen der Formel (I-a) sind erfindungsgemäße Verbindungen und können nach Verfahren (a) hergestellt werden.

Die zur Durchführung des erfindungsgemäßen Verfahrens (b) weiterhin als Ausgangsstoffe benötigten Halogenide sind durch die Formel (IV) allgemein definiert. In dieser Formel (IV) steht R^{1-B} bevorzugt, besonders bevorzugt, ganz besonders bevorzugt bzw. insbesondere bevorzugt für diejenigen Bedeutungen, die bereits oben für den Rest R¹ als bevorzugt, besonders bevorzugt usw. angegeben wurden, wobei R^{1-B} niemals für Wasserstoff steht. Hal steht für Chlor, Brom oder Iod.

Halogenide der Formel (IV) sind bekannt.

Als Verdünnungsmittel zur Durchführung des erfindungsgemäßen Verfahrens (a) kommen alle inerten organischen Lösungsmittel in Betracht. Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; deren Gemische mit Wasser oder reines Wasser.

Das erfindungsgemäße Verfahren (a) wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie z.B. Natriumhydrid, Natriumamid, Lithiumdiisopropylamid, Natrium-methylat, Natrium-ethylat, Kalium-tert.-butylat, Natriumhydroxid, Kaliumhydroxid, Natriumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Das erfindungsgemäße Verfahren (a) wird gegebenenfalls in Gegenwart eines geeigneten Kupplungsreagenzes durchgeführt (wenn X⁵ für Hydroxy steht). Als solche kommen alle üblichen Carbonylaktivatoren infrage. Hierzu gehören vorzugsweise N-[3-(Dimethylamino)propyl]-N'-ethyl-carbodiimide-hydrochlorid, N,N'-Di-sec-butyl-carbodiimid, NN'-Dicyclohexylcarbodiimid, N,N'-Diisopropylcarbodiimid, 1-(3-(Dimethylamino)propyl)-3-ethyl-carbodiimid-methiodid, 2-Bromo-3-ethyl-4-methyl-thiazolium-tetrafluoroborat, N,N-Bis[2-oxo-3-oxazolidinyl]phosphorodiamidic chloride, Chlor-tripyrrolidino-phosphonium-hexafluorphosphat, Brom-tripyrrolidino-phosphonium-hexafluorphosphat, O-(1H-Benzotriazol-1-yloxy)tris(dimethylamino)phosphonium-hexafluorphospha, NNN',N'-Bis(tetramethylen)chloruronium-tetrafluorborat, O-(1H-Benzatriazol-1-yl)-N,N,N',N'-tetramethyluronium-hexafluorphosphat, O-(1H-Benzotriazol-1-yl)-N,N,N',N'-bis(tetramethylen)uronium-hexafluorphosphat, O-(1H-Benzotriazol-1-yl)-N,N,N',N'-bis(tetramethylen)uronium-tetrafluorborat, O-(7-Azabenzotriazol-1-yl)-N,N,N,N-tetramethyhuronium hexafluorphosphat und 1-Hydroxybenzotriazol. Diese Reagenzien können separat aber auch in Kombination eingesetzt werden.

Die Reaktionstemperaturen können bei der Durchführung des erfindungsgemäßen Verfahrens (a) in einem größeren Bereich variiert werden. Im Allgemeinen arbeitet man bei Temperaturen von 0°C bis 150°C, vorzugsweise bei Temperaturen von 20°C bis 110°C.

Zur Durchführung des erfindungsgemäßen Verfahrens (a) zur Herstellung der Verbindungen der Formel (I) setzt man pro Mol des Cajbonsäurehalogenids der Formel (II) im Allgemeinen 0,2 bis 5 Mol, vorzugsweise 0,5 bis 2 Mol an Anilinderivat der Formel (III) ein. Die Aufarbeitung erfolgt nach üblichen Methoden.

Als Verdünnungsmittel zur Durchführung des erfindungsgemäßen Verfahrens (b) kommen alle inerten organischen Lösungsmittel in Betracht. Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlormethan, Dichlorethan oder Trichlorethan; Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Das erfindungsgemäße Verfahren (b) wird in Gegenwart einer Base durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie z.B. Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Caesiumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen von 0°C bis 150°C, vorzugsweise bei Temperaturen von 20°C bis 110°C.

Zur Durchführung des erfindungsgemäßen Verfahrens (b) zur Herstellung der Verbindungen der Formel (I) setzt man pro Mol des Carboxamids der Formel (I-a) im Allgemeinen 0,2 bis 5 Mol, vorzugsweise 0,5 bis 2 Mol an Halogenid der Formel (IV) ein.

Als Verdünnungsmittel zur Durchführung des erfindungsgemäßen Verfahrens (c) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Die Reaktionstemperaturen können bei der Durchführung des erfindungsgemäßen Verfahrens (c) in einem größeren Bereich variiert werden. Im Allgemeinen arbeitet man bei Temperaturen von 100°C bis 250°C, vorzugsweise bei Temperaturen von 140°C bis 200°C.

Das erfindungsgemäße Verfahren (c) wird gegebenenfalls in Gegenwart einer geeigneten Säure durchgeführt. Als solche kommen alle üblichen anorganischen oder organischen Säuren infrage. Vorzugsweise verwendbar sind Salzsäure, Schwefelsäure, Flusssäure, Phosphorsäure, p-Toluolsulfonsäure, Methansulfonsäure, Trifluoressigsäure oder Trichloressigsäure. Besonders bevorzugt verwendet man Salzsäure, Schwefelsäure, p-ToluoIsulfonsäure oder Trifluoressigsäure, ganz besonders bevorzugt p-Toluolsulfonsäure. Die eingesetzte Säure kann gegebenenfalls in einer Mischung mit Wasser eingesetzt werden.

Zur Durchführung des erfindungsgemäßen Verfahrens (c) zur Herstellung der Verbindungen der Formel (III-a) setzt man pro Mol des Phenylacetonitrils der Formel (V) im Allgemeinen 1 bis 10 Mol, vorzugsweise 1 bis 3 Mol an Tris(formamino)methan und 1 bis 100 Mol, vorzugsweise 3 bis 10 Mol Formamid ein. Die Aufarbeitung erfolgt nach üblichen Methoden.

Die erfindungsgemäßen Verfahren (a), (b) und (c) werden im Allgemeinen unter Normaldruck durchgeführt. Es ist jedoch auch möglich, unter erhöhtem oder vermindertem Druck - im Allgemeinen zwischen 0,1 bar und 10 bar - zu arbeiten.

Die erfindungsgemäßen Stoffe weisen eine starke mikrobizide Wirkung auf und können zur Bekämpfung von unerwünschten Mikroorganismen, wie Pilze und Bakterien, im Pflanzenschutz und im Materialschutz eingesetzt werden.

Fungizide lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Erkrankungen, hervorgerufen durch Erreger des Echten Mehltaus wie z.B.
Blumeria-Arten, wie beispielsweise Blumeria graminis;
Podosphaera-Arten, wie beispielsweise Podosphaera leucotricha;
Sphaerotheca-Arten, wie beispielsweise Sphaerotheca fuliginea;
Uncinula-Arten, wie beispielsweise Uncinula necator;

Erkrankungen, hervorgerufen durch Erreger von Rostkrankheiten wie z.B.
Gymnosporangium-Arten, wie beispielsweise Gymnosporangium sabinae
Hemileia-Arten, wie beispielsweise Hemileia vastatrix;
Phakopsora-Arten, wie beispielsweise Phakopsora pachyrhizi und Phakopsora meibomiae;
Puccinia-Arten, wie beispielsweise Puccinia recondita oder Puccinia graminis;
Uromyces-Arten, wie beispielsweise Uromyces appendiculatus;

Erkrankungen, hervorgerufen durch Erreger der Gruppe der Oomycete wie z.B.
Bremia-Arten, wie beispielsweise Bremia lactucae;
Peronospora-Arten, wie beispielsweise Peronospora pisi oder P. brassicae;
Phytophthora-Arten, wie beispielsweise Phytophthora infestans;
Plasmopara-Arten, wie beispielsweise Plasmopara viticola;
Pseudoperonospora-Arten, wie beispielsweise Pseudoperonospora humuli oder
Pseudoperonospora cubensis;
Pythium-Arten, wie beispielsweise Pythium ultimum;

Blattfleckenkrankheiten und Blattwelken, hervorgerufen durch z.B.
Alternaria-Arten, wie beispielsweise Alternaria solani;
Cercospora-Arten, wie beispielsweise Cercospora beticola;
Cladosporium-Arten, wie beispielsweise Cladosporium cucumerinum;
Cochliobolus-Arten, wie beispielsweise Cochliobolus sativus
(Konidienform: Drechslera, Syn: Helminthosporium);
Colletotrichum-Arten, wie beispielsweise Colletotrichum lindemuthanium;
Cycloconium-Arten, wie beispielsweise Cycloconium oleaginum;
Diaporthe-Arten, wie beispielsweise Diaporthe citri;
Elsinoe-Arten, wie beispielsweise Elsinoe fawcettii;
Gloeosporium-Arten, wie beispielsweise Gloeosporium laeticolor;
Glomerella-Arten, wie beispielsweise Glomerella cingulata;
Guignardia-Arien, wie beispielsweise Guignardia bidwelli;
Leptosphaeria-Arten, wie beispielsweise Leptosphaeria maculans;
Magnaporthe-Arten, wie beispielsweise Magnaporthe grisea;
Mycosphaerella-Arten, wie beispielsweise Mycosphaerella graminicola und Mycosphaerella fijiensis;
Phaeosphaeria-Arten, wie beispielsweise Phaeosphaeria nodorum;
Pyrenophora-Arten, wie beispielsweise Pyrenophora teres;
Ramularia-Arten, wie beispielsweise Ramularia collo-cygni;
Rhynchosporium-Arten, wie beispielsweise Rhynchosporium secalis;
Septoria-Arten, wie beispielsweise Septoria apii;
Typhula-Arten, wie beispielsweise Typhula incarnata;
Venturia-Arten, wie beispielsweise Venturia inaequalis;

Wurzel- und Stengelkrankheiten, hervorgerufen durch z.B.
Corticium-Arten, wie beispielsweise Corticium graminearum;
Fusarium-Arten, wie beispielsweise Fusarium oxysporum;
Gaeumannomyces-Arten, wie beispielsweise Gaeumannomyces graminis;
Rhizoctonia-Arten, wie beispielsweise Rhizoctonia solani;
Tapesia-Arten, wie beispielsweise Tapesia acuformis oder Tapesia yallundae;
Thielaviopsis-Arten, wie beispielsweise Thielaviopsis basicola;

Ähren- und Rispenerkrankungen (inklusive Maiskolben), hervorgerufen durch z.B.
Alternaria-Arten, wie beispielsweise Alternaria spp.;
Aspergillus-Arten, wie beispielsweise Aspergillus flavus;
Cladosporium-Arten, wie beispielsweise Cladosporium cladosporioides;
Claviceps-Arten, wie beispielsweise Claviceps purpurea;
Fusarium-Arten, wie beispielsweise Fusarium culmorum;
Gibberella-Arten, wie beispielsweise Gibberella zeae;
Monographella-Arten, wie beispielsweise Monographella nivalis;

Erkrankungen, hervorgerufen durch Brandpilze wie z.B.
Sphacelotheca-Arten, wie beispielsweise Sphacelotheca reiliana;
Tilletia-Arten, wie beispielsweise Tilletia caries;
Urocystis-Arten, wie beispielsweise Urocystis occulta;
Ustilago-Arten, wie beispielsweise Ustilago nuda;
Fruchtfäule hervorgerufen durch z.B.
Aspergillus-Arten, wie beispielsweise Aspergillus flavus;
Botrytis-Arten, wie beispielsweise Botrytis cinerea;
Penicillium-Arten, wie beispielsweise Penicillium expansum und Penicillium purpurogenum;
Sclerotinia-Arten, wie beispielsweise Sclerotinia sclerotiorum;
Verticilium-Arten, wie beispielsweise Verticilium alboatrum;

Samen- und bodenbürtige Fäulen und Welken, sowie Sämlingserkrankungen, hervorgerufen durch z.B.
Fusarium-Arten, wie beispielsweise Fusarium culmorum;
Phytophthora Arten, wie beispielsweise Phytophthora cactorum;
Pythium-Arten, wie beispielsweise Pythium ultimum;
Rhizoctonia-Arten, wie beispielsweise Rhizoctonia solani;
Sclerotium-Arten, wie beispielsweise Sclerotium rolfsii;

Krebserkrankungen, Gallen und Hexenbesen, hervorgerufen durch z.B.
Nectria-Arten, wie beispielsweise Nectria galligena;

Welkeerkrankungen hervorgerufen durch z.B.
Monilinia-Arten, wie beispielsweise Monilinia laxa;

Deformationen von Blättern, Blüten und Früchten, hervorgerufen durch z.B.
Taphrina-Arten, wie beispielsweise Taphrina deformans;

Degenerationserkrankungen holziger pflanzen, hervorgerufen durch z.B.
Esca-Arten, wie beispielsweise Phaeomoniella chlamydospora und Phaeoacremonium aleophilum und Fomitiporia mediterranea;
Blüten- und Samenerkrankungen, hervorgerufen durch z.B.
Botrytis-Arten, wie beispielsweise Botrytis cinerea,

Erkrankungen von Pflanzenknollen, hervorgerufen durch z.B.
Rhizoctonia-Arten, wie beispielsweise Rhizoctonia solani;
Helminthosporium-Arten, wie beispielsweise Helminthosporium solani;

Erkrankungen, hervorgerufen durch bakterielle Erreger wie z.B.
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora.

Bevorzugt können die folgenden Krankheiten von Soja-Bohnen bekämpft werden:
Pilzkrankheiten an Blättern, Stängeln, Schoten und Samen verursacht durch z.B.
   Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi), Scab (Sphaceloma glycines), Stemphylium Leaf Blight (Stemphylium botryosum), Target Spot (Corynespora cassiicola).
Pilzkrankheiten an Wurzeln und der Stängelbasis verursacht durch z.B.
   Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmospora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen von 1 bis 28 Tage, vorzugsweise 1 bis 14 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie z.B. gegen Puccinia-Arten und von Krankheiten im Wein-, Obst- und Gemüseanbau, wie z.B. gegen Botrytis-, Venturia- oder Alternaria-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.
Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Spritzen, Vernebeln, Streuen, Aufstreichen und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen infrage:
Fungizide:
   1) Inhibitoren der Nukleinsäuresynthese: z.B. Benalaxyl, Benalaxyl-M, Bupirimate, Clozylacon, Dimethirimol, Ethirimol, Furalaxyl, Hymcxazol, Mefenoxam, Metalaxyl, Metalaxyl-M, Ofurace, Oxadixyl, Oxolinic acid;
   2) Inhibitoren von Mitose und Zellteilung: z.B. Benomyl, Carbendazim, Diethofencarb, Ethaboxam, Fuberidazole, Pencycuron, Thiabendazole, Thiophanate-methyl, Zoxamide;
   3) Inhibitoren der Respiration (Atmungsketten-Inhibitoren):
      3.1) Inhibitoren am Komplex I der Atmungskette: z.B. Diflumetorim;
      3.2) Inhibitoren am Komplex II der Atmungskette: z.B. Boscalid/Nicobifen, Carboxin, Fenfuram, Flutolanil, Furametpyr, Furmecyclox, Mepronil, Oxycarboxin, Penthiopyrad, Thifluzamide;
      3.3) Inhibitoren am Komplex III der Atmungskette: z.B. Amisulbrom, Azoxystrobin, Cyazofamid, Dimoxystrobin, Enestrobin, Farnoxadone, Fenamidone, Fluoxastrobin, Kresoxim-methyl, Metominostrobin, Orysastrobin, Picoxystrobin, Pyraclostrobin, Trifloxystrobin;
   4) Entkoppler: z.B. Dinocap, Fluazinam, Meptyldinocap;
   5) Inhibotoren der ATP Produktion: z.B. Fentin acetate, Fentin chloride, Fentin hydroxide, Silthiofam;
   6) Inhibitoren der Aminosäure- und Protein-Biosynthese: z.B. Andoprim, Blasticidin-S, Cyprodinil, Kasugamycin, Kasugamycin hydrochloride hydrate, Mepanipyrim, Pyrimethanil;
   7) Inhibitoren der Signaltransduktion: z.B. Fenpiclonil, Fludioxonil, Quinoxyfen;
   8) Inhibitoren der Lipid- und Membran-Synthese: z.B. Biphenyl, Chlozolinate, Edifenphos, Iodocarb, Iprobenfos, Iprodione, Isoprothiolane, Procymidone, Propamocarb, Propamocarb hydrochloride, Pyrazophos, Tolclofos-methyl, Vinclozolin;
   9) Inhibitoren der Ergosterol-Biosynthese: z.B. Aldimorph, Azaconazole, Bitertanol, Bromuconazole, Cyproconazole, Diclobutrazole, Difenoconazole, Diniconazole, Diniconazole-M, Dodemorph, Dodemorph acetate, Epoxiconazole, Etaconazole, Fenarimol, Fenbuconazole, Fenhexamid, Fenpropidin, Fenpropimorph, Fluquinconazole, Flurprimidol, Flusilazole, Flutriafol, Furconazole, Furconazole-cis, Hexaconazole, Imazalil, Imazalil sulfate, Imibenconazole, Ipconazole, Metconazole, Myclobutanil, Naftifine, Nuarimol, Oxpoconazole, Paclobutrazol, Pefurazoate, Penconazole, Prochloraz, Propiconazole, Prothioconazole, Pyributicarb, Pyrifenox, Simeconazole, Spiroxamine, Tebuconazole, Terbinafine, Tetraconazole, Triadimefon, Triadimenol, Tridemorph, Triflumizole, Triforine, Triticonazole, Uniconazole, Viniconazole, Voriconazole;
   10) Inhibitoren der Zellwandsynthese: z.B. Benthiavalicarb, Dimethomorph, Flumorph, Iprovalicarb, Polyoxins, Polyoxorim, Validamycin A;
   11) Inhibitoren der Melanin-Biosynthese: z.B. Carpropamid, Diclocymet, Fenoxanil, Phthalide, Pyroquilon, Tricyclazole;
   12) Resistenzinduktoren: z.B. Acibenzolar-S-methyl, Probenazole, Tiadinil;
   13) Verbindungen mit Multisite-Aktivtät: z.B. Bordeaux Mixture, Captafol, Captan, Chlorothalonil, Copper naphthenate, Copper oxide, Copper oxychloride, Kupferzubereitungen wie z.B. Kupferhydroxid, Kupfersulfat, Dichlofluanid, Dithianon, Dodine, Dodine free base, Ferbam, Fluorofolpet, Folpet, Guazatine, Guazatine acetate, Iminoctadine, Iminoctadine albesilate, Iminoctadine triacetate, Mancopper, Mancozeb, Maneb, Metiram, Metiram Zinc, Oxine-Copper, Propineb, Sulphur und Schweferlzubercitungen wie z.B. Calcium polysulphide, Thiram, Tolylfluanid, Zineb, Ziram;
   14) eine Verbindung aus der folgenden Liste: (2E)-2-(2-{[6-(3-Chlor-2-methylphenoxy)-5-fluorpyrimidin-4-yl]oxy}phenyl)-2-(methoxyimino)-N-methylacetamid, (2E)-2-{2-[({[(1E)-1-(3-{[(E)-1-Fluor-2-phenylvinyl]oxy}phenyl)ethyliden]amino}oxy)methyl]phenyl}-2-(methoxyimino)-N-methylacetamid, 1-(4-Chlorphenyl)-2-(1H-1,2,4-triazol-1-yl)cycloheptanol, 1-[(4-Methoxyphenoxy)methyl]-2,2-dimethylpropyl-1H-imidazol-1-carboxylat, 2-(4-Chlorphenyl)-N-{2-[3-methoxy-4-(prop-2-yn-1-yloxy)phenyl]ethyl}-2-(prop-2-yn-1-yloxy)acetamid, 2,3,5,6-Tetrachlor-4-{methylsulfonyl)pyridin, 2-Butoxy-6-iod-3-propyl-4H-chromen-4-on, 2-Chlor-N-(1,13-trimethyl-2,3-dihydro-1H-inden-4-yl)niconamid, 2-Phenylphenol und Salze davon, 3,4,5-Trichlorpyridin-2,6-dicarbonitril, 3,4-Dichlor-N-(2-cyanophenyl)isothiazol-5-carboxamid, 3-[5-(4-Chlorphenyl)-2,3-dimethylisoxazolidin-3-yl]pyridin, 5-Chlor-6-(2,4,6-trifluorophenyl)-N-[(1R)-1,2,2-trimethylpropyl][1,2,4]triazolo[1,5-a]pyrimidin-7-amin, 5-Chlor-7-(4-methypiperidin-1-yl)-6-(2,4,6-thflorphenyl)[1,2,4]triazolo[1,5-a]pyrimidin, 5-Chlor-N-[(1R)-1,2-dimethylpropyl]-6-(2,4,6-trifluorphenyl)[1,2,4 ]triazolo[1,5-a]pyrimidin-7-amin, 8-Hydroxyquinolinsulfat, Benthiazole, Bethoxazin, Capsimycin, Carvone, Chinomethionat, Cufraneb, Cyflufenamid, Cymoxanil, Dazomet, Debacarb, Dichlorophen, Diclomezine, Dicloran, Difenzoquat, Difenzoquat Methylsulphate, Diphenylamine, Ferimzone, Flumetover, Fluopicolide, Fluoroimide, Flusulfamide, Fosetyl-Aluminium, Fosetyl-Calcium, Fosetyl-Sodium, Hexachlorobenzene, Irumamycin, Methasulfocarb, Methyl (2-chlor-5-{(1E)-N-[(6-methylpyridin-2-yl)methoxy]ethanimidoyl}benzyl)carbamat, Methyl (2E)-2-{2-[({cyclopropyl[(4-methoxyphenyl)imino]methyl}thio)methyl]phenyl}-3-methoxyacryat, Methyl 1-(2,2-dimethyl-2,3-dihydro-1H-inden-1-yl)-1H-imidazol-5-carboxylat, Methyl 3-(4-chlorphenyl)-3-{[N-(isopropoxycarbonyl)valyl]amino}propanoat, Methyl isothiocyanate, Metrafenone, Mildiomycin, N-(3',4'-Dichlor-5-fluorbiphenyl-2-yl)-3-(difluormethyl)-1-methyl-1H-pyazol-4-carboxamid, N-(3-Ethyl-3,5,5-trimethylcyclohexyl)-3-(formylamino)-2-hydroxybenzamid, N-(4-Chlor-2-nitrophenyl)-N-ethyl-4-methylbenzensulfonamid, N-[(5-Brom-3-chlorpyridin-2-yl)methyl]-2,4-dichlomicotinamid, N-[1-(5-Brom-3-chlorpyridin-2-yl)ethyl]-2,4-dichlomicotinamid, N-[1-(5-Brom-3-chlorpyridin-2-yl)ethyl]-2-fluor-4-iodnicotinamid, N-[2-(4-{[3-(4-Chlorphenyl)prop-2-yn-1-yl]oxy}-3-methoxyphenyl)ethyl]-N²-(methylsulfonyl)valinamid, N-{(Z)-[(Cyclopropylmethoxy)imino][6-(difluormethoxy)-2,3-difluorphenyl]methyl}-2-phenylacetamid, N-{2-[3-Chlor-5-(trifluormethyl)pyridin-2-yl]ethyl}-2-(trifluormethyl)benzamid, Natamycin, Nickel Dimethyldithiocarbamate, Nitrothal-isopropyl, O-{1-[(4-Methoxyphenoxy)methyl]-2,2-dimethylpropyl} 1H-Imidazol-1-carbothioat Octhilnone, Oxamocarb, Oxyfenthim, Pentachlorophenol und Salze, Phophorsäure und ihre Salze, Piperalin, Propamocarb Fosetylate, Propanosine-Sodium, Proquinazid, Pyrrolnitrine, Quintozene, Tecloftalam, Tecnazene, Triazoxide, Trichlamide, Zarilamid.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

*1. Acetylcholinesterase (AChE) Inhibitoren*
   1.1 Carbamate (z.B. Alanycarb, Aldicarb, Aldoxycarb, Allyxycarb, Aminocarb, Azamethiphos, Bendiocarb, Benfuracarb, Bufencarb, Butacarb, Butocarboxim, Butoxycarboxim, Carbaryl, Carbofuran, Carbosulfan, Chloethocarb, Coumaphos, Cyanofenphos, Cyanophos, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Triazamate, Trimethacarb, XMC, Xylylcarb)
   1.2 Organophosphate (z.B. Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S--methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazale, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/-ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion)
*2. Natrium-Kanal-Modulatoren* / *Spannungsabhängige Natrium-Kanal-Blocker*
   2.1 Pyrethroide (z.B. Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, DDT, Dehamethrin, Empenthrin (1R-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvatinate, Fubfenprox, Gamma-Cyhalothrin, Imiprothrin, Kadethrin, Lambda-Cyhalothrin, Metofluthrin, Permethrin (cis-, trans-), Phenothrin (IR-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (IR-isomer), Tralomethrin, Transfluthrin, ZXI 8901, Pyrethrins (pyrethrum))
   2.2 Oxadiazine (z.B. Indoxacarb)
*3. Acetylcholin-Rezeptor-Agonisten*/*-Antagonisten*
   3.1 Chloronicotinyle/Neonicotinoide (z.B. Acetamiprid, Clothianidin, Dinotefuran, Imidacloprid, Nitenpyram, Nithiazine, Thiacloprid, Thiamethoxam)
   3.2 Nicotine, Bensultap, Cartap
*4. Acetylcholin-Rezeptor Modulatoren*
   4.1 Spinosyne (z.B. Spinosad)
*5. GABA-gesteuerte Chlorid Kanal Antagonisten*
   5.1 Cyclodiene Organochlorine (z.B. Camphechlor, Chlordane, Endosulfan, Gamma-HCH, HCH, Heptachlor, Lindane, Methoxychlor
   5.2 Fiprole (z.B. Acetoprole, Ethiprole, Fipronil, Vaniliprole)
*6. Chlorid-Kanal-Aktivatoren*
   6.1 Mectine (z.B. Abamectin, Avermectin, Emamectin, Emamectin-benzoate, Ivermectin, Milbemectin, Milbemycin)
*7. Juvenilhormon-Mimetika*
   (z.B. Diofenolan, Epofenonane, Fenoxycarb, Hydroprene, Kinoprene, Methoprene, Pyriproxifen, Triprene)
*8. Ecdysonagonisten*/*disruptoren*
   8.1 Diacylhydrazine (z.B. Chromafenozide, Halofenozide, Methoxyfenozide, Tebufenozide)
*9. Inhibitoren der Chitinbiosynthese*
   9.1 Benzoylharnstoffe (z.B. Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron)
   9.2 Buprofezin
   9.3 Cyromazine
*10*. *Inhibitoren der oxidativen Phosphorylierung, ATP-Disruptoren*
   10.1 Diafenthiuron
   10.2 Organotine (z.B. Azocyclotin, Cyhexatin, Fenbutatin-oxide)
*11*. *Entkoppler der oxidativen Phoshorylierung durch Unterbrechung des H-Protongradienten*
   11.1 Pyrrole (z.B. Chlorfenapyr)
   11.2 Dinitrophenole (z.B. Binapacyrl, Dinobuton, Dinocap, DNOC)
*12. Site-I-Elektronentransportinhibitoren*
   12.1 METI's (z.B. Fenazaquin, Fenpyroximate, Pyrimidifen, Pyridaben, Tebufenpyrad, Tolfenpyrad)
   12.2 Hydramethylnone
   12.3 Dicofol
*13. Site-II-Elektronentransportinhibitoren*
   13.1 Rotenone
*14. Site-III-Elektronentransportinhibitoren*
   14.1 Acequinocyl, Fluacrypyrim
*15. Mikrobielle Disruptoren der Insektendarmmembran*
   Bacillus thuringiensis-Stämme
*16. Inhibitoren der Fettsynthese*
   16.1 Tetronsäuren (z.B. Spirodiclofen, Spiromesifen)
   16.2 Tetramsären [z.B. 3-(2,5-Dimethylphenyl)-8-methoxy-2-oxo-1-azaspiro[4.5]dec-3-en-4-yl ethyl carbonate (alias: Carbonic acid, 3-(2,5-dimethylphenyl)-8-methoxy-2-oxo-1-azaspiro[4.5]dec-3-en-4-yl ethyl ester, CAS-Reg.-No.: 382608-10-8) and Carbonic acid, cis-3-(2,5-dimethylphenyl)-8-methoxy-2-oxo-1-azaspiro[4.5]dec-3-en-4-yl ethyl ester (CAS-Reg.-No.: 203313-25-1)]
*17. Carboxamide*
   (z.B. Flonicamid)
*18. Oktopaminerge Agonisten*
   (z.B. Amitraz)
*19. Inhibitoren der Magnesium-stimulierten ATPase*
   (z.B. Propargite)
*20. Agonisten des Ryanodin-Rezeptors,*
   20.1 Benzoesäuredicarboxamide [z.B. N²-[1,1-Dimethyl-2-(methylsulfonyl)ethyl]-3-iod-N¹-[2-methyl-4-[1,2,2,2-tetrafluor-1-(trifluormethyl)ethyl]phenyl]-1,2-benzenedicarboxamide (CAS-Reg.-No.: 272451-65-7), Flubendiamide]
   20.2 Anthranilamide (z.B. DPX E2Y45 = 3-Brom-N-{4-cHor-2-methyl-6-[(methylamino)carbonyl]-phenyl}-1-(3-chlorpyridin-2-yl)-1H-pyrazol-5-carboxamid)
*21. Nereistoxin-Analoge*
   (z.B. Thiocyclam hydrogen oxalate, Thiosultap-sodium)
*22. Biologika, Hormone oder Pheromone.*
   (z.B. Azadirachtin, Bacillus spec., Beauveria spec., Codlemone, Metarrhizium spec., Paecilomyces spec., Thuringiensin, Verticillium spec.)
*23. Wirkstoffe mit unbekannten oder nicht spezifischen Wirkmechanismen*
   23.1 Begasungsmittel (z.B. Aluminium phosphide, Methyl bromide, Sulfuryl fluoride)
   23.2 Selektive Fraßhemmer (z.B. Cryolite, Flonicamid, Pymetrozine)
   23.3 Milbenwachstumsinhibitoren (z.B. Clofentezine, Etoxazole, Hexythiazox)
   23.4 Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyrafluprole, Pyridalyl, Pyriprole, Sulfluramid, Tetradifon, Tetrasul, Triarathene, Verbutin, ferner die Verbindung 3-Methyl-phenyl-propylcarbamat (Tsumacide Z), die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO 96/37494, WO 98/25923), sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren, Safener bzw. Semiochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, weiter entwickeltes Wurzelsystem, höhere Beständigkeit der Pflanzenart bzw. Pflanzensorte, gesteigertes Wachstum der Schösslinge, höhere Pflanzenvitalität erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, größere Früchte, höhere Pflanzengröße, grünere Blattfarbe, frühere Blüte, höhere Qualität und/oder höherer Ernährungswert der Ernteprodukte, höhere Zuckerkonzentration in den Früchten, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im Folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, z.B. Imidazolinonen, Sulfonylharnstoffen, Glyphosate oder Phosphinoticin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucoton® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Herstellung und die Verwendung der erfindungsgemäßen Wirkstoffe geht aus den folgenden Beispielen hervor.

### Herstellungsbeispiele

### Beispiel 1

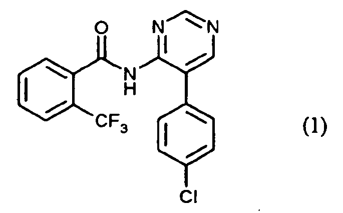

0,2 g (1,0 mmol) 5-(4-Chlorphenyl)-4-pyrimidinylamin werden in 6 ml Tetrahydrofuran gelöst und mit 0,2 g (2,0 mmol) Triethylamin versetzt. Bei 0°C werden 0,25 g (1,2 mmol) 2-Trifluormethylbenzoesäurechlorid zugegeben. Die Reaktionslösung wird für 16 h bei 60°C gerührt. Zur Aufarbeitung wird filtriert und aufkonzentriert. Das Rohprodukt wird mittels Säulenchromatographie (Cyclohexan/Essigsäureethylester 2:1) gereinigt. Man erhält 0,06 g (15% der Theorie) an *N-*[5-(4-Chlorphenyl)-4-pyrimidinyl]-2-(trifluormethyl)benzamid mit dem LogP (pH 2,3) = 2,57.

### Beispiel 2

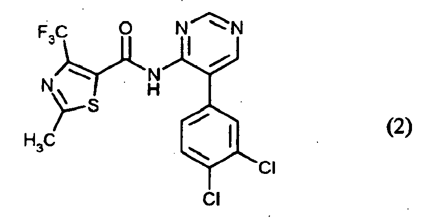

0,21 g (1,0 mmol) 2-Methyl-4-(trifluormethyl)-1,3-thiazol-5-carbonsäure, 0,49 g (2,4 mmol) 1,3-Dicyclohexylcarbodiimid und 0,14 g (1,0 mmol) 1-Hydroxybenzotriazol werden in 15 ml Dichlormethan suspendiert. Nach fünf Minuten werden 0,24 g (1,0 mmol) 5-(3,4-Dichlorphenyl)-4-pyrimidinylamin zugegeben. Die Reaktionslösung wird für 8 h bei Raumtemperatur und anschließend für 16 h bei 40°C gerührt. Zur Aufarbeitung wird das Reaktionsgemisch filtriert, mit gesättigter Natriumhydrogencarbonatlösung gewaschen, über Natriumsulfat getrocknet und aufkonzentriert. Säulenchromatographie (Cyclohexan/Essigsäureethylester 2:1) liefert 0,15 g (34 % der Theorie) an *N*-[5-(3,4-Dichlorphenyl)-4-pyrimidinyl]-2-methyl-4-(trifluormethyl)-1,3-thiazol-5-carboxamid mit dem LogP (pH 2,3) = 2,85.

Analog den Beispielen 1 und 2 sowie entsprechend den allgemeinen Beschreibungen der erfindungsgemäßen Verfahren können die in der folgenden Tabelle 1 genannten Verbindungen der Formel (I) erhalten werden.

**Tabelle 1**

| | | | | | | | | |
|---|---|---|---|---|---|---|---|---|
| 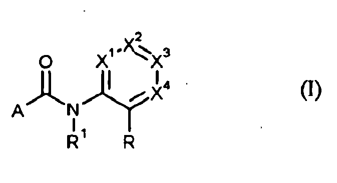 | | | | | | | | |

| **Nr.** | **R** | **R¹** | **X¹** | **X²** | **X³** | **X⁴** | **A** | **LogP (pH 2,3)** |
|---|---|---|---|---|---|---|---|---|
| 3 | 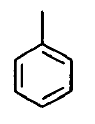 | H | N | CH | N | CH | 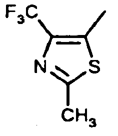 | 2,29 |
| 4 | 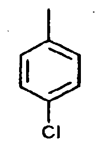 | H | N | CH | N | CH | 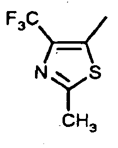 | 2,49 |
| 5 | 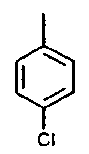 | H | N | CH | N | CH | 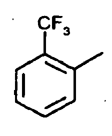 | 1,79 |
| 6 | 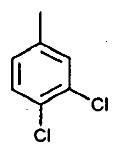 | H | N | CH | N | CH | 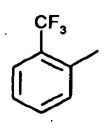 | 2,92 |
| 7 | 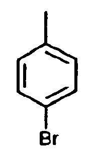 | H | N | CH | N | CH | 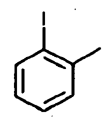 | 2,61 |
| 8 | 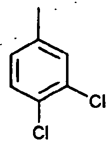 | H | N | CH | N | CH | 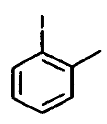 | 2,89 |
| 9 | 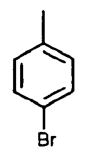 | H | N | CH | N | CH | 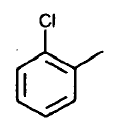 | 2,46 |
| 10 | 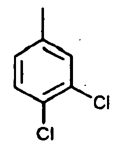 | H | N | CH | N | CH | 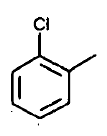 | 2,73 |
| 11 | 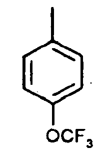 | H | N | CH | N | CH | 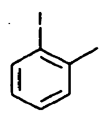 | 2,85 |
| 12 | 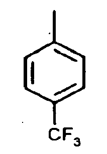 | 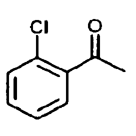 | N | CH | N | CH | 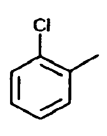 | 4,12 |
| 13 | 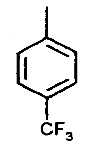 | H | N | CH | N | CH | 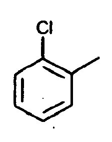 | 2,62 |
| 14 | 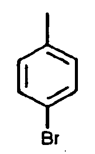 | 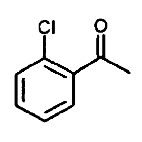 | N | CH | N | CH | 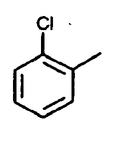 | 2,73 |
| 15 | 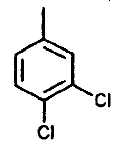 | 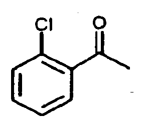 | N | CH | N | CH | 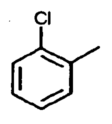 | 2,46 |
| 16 | 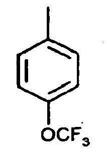 | 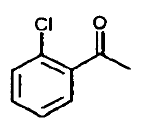 | N | CH | N | CH | 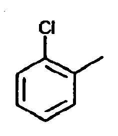 | 4,25 |
| 17 | 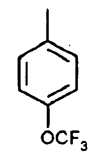 | H | N | CH | N | CH | 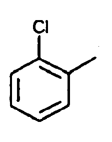 | 2,73 |
| 18 | 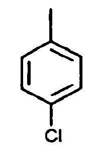 | 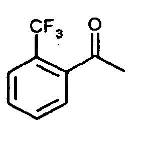 | N | CH | N | CH | 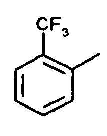 | 4,29 |
| 19 | 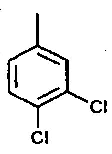 | H | N | CH | N | CH | 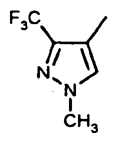 | 2,46 |
| 20 | 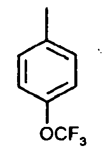 | H | N | CH | N | CH | 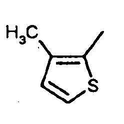 | |

### Herstellung von Ausgangsstoffen der Formel (III)

### Beispiel (III-1)

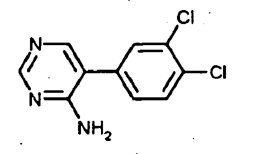

15,0 g (0,08 Mol) 3,4-Dichlorbenzylcyanid werden mit 23,4 g (0,016 Mol) Tris(formamino)methan und 1,4 g (0,008 Mol) p-Toluolsulfonsäure in 15,5 ml Formamid für 10 h bei 170-180°C gerührt. Zur Aufarbeitung wird mit 10%iger Salzsäure angesäuert, Aktivkohle zugegeben und 5 min gerührt. Nach Filtration wird das Filtrat mit 10%iger NaOH basisch gestellt. Der entstehende weiße Niederschlag wird abgesaugt, in einem Gemisch aus 5 % Methanol in Chloroform aufgenommen und über Celite filtriert. Man erhält 8,2 g (42 % der Theorie) an 5-(3,4-Dichlorophenyl)-4-pyrimidinylamin mit dem LogP (pH 2,3) = 0,87.

Analog Beispiel (III-1) sowie entsprechend den allgemeinen Beschreibungen der erfindungsgemäßen Verfahren können die in der folgenden Tabelle 2 genannten Verbindungen der Formel (III) erhalten werden.

**Tabelle 2**

| | | | | | | | |
|---|---|---|---|---|---|---|---|
| 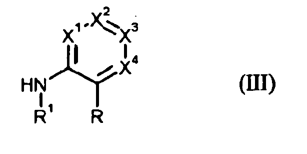 | | | | | | | |

| **Nr.** | **R** | **R¹** | **X¹** | **X¹** | **X³** | **X⁴** | **LogP (pH 2,3)** |
|---|---|---|---|---|---|---|---|
| III-2 | 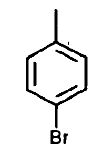 | H | N | CH | N | CH | 0,56 |
| III-3 | 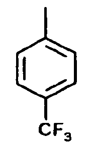 | H | N | CH | N | CH | 0,80 |
| III-4 | 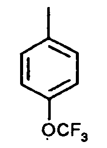 | H | N | CH | N | CH | 1,00 |

Die Bestimmung der in den voranstehenden Tabellen und Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C.

Die Bestimmung erfolgt im sauren Bereich bei pH 2.3 mit 0,1 % wässriger Phosphorsäure und Acetonitril als Eluenten; linearer Gradient von 10 % Acetonitril bis 90 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele

### Beispiel A

### Sphaerotheca - Test (Gurke) / protektiv

| | | |
|---|---|---|
| Lösungsmittel: | 49 | Gewichtsteile N,N-Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Gurkenpflanzen mit der Wirkstoffzubereitung in der angegebenen Aufwandmenge. 1 Tag nach der Behandlung werden die Pflanzen mit einer Sporensuspension von Sphaerotheca fuliginea inokuliert. Anschließend werden die Pflanzen in einem Gewächshaus bei 70 % relativer Luftfeuchtigkeit und einer Temperatur von 23°C aufgestellt. 7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle A**

| **Sphaerotheca - Test (Gurke) / protektiv** | | | |
|---|---|---|---|
| Wirkstoff Erfindungsgemäß | | Aufwandmenge an Wirkstoff in ppm | Wirkungsgrad in % |
| (1) | 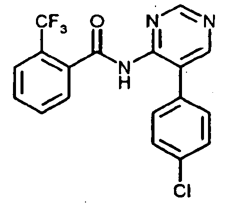 | 500 | 78 |
| (7) | 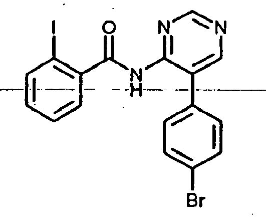 | 500 | 95 |
| (8) | 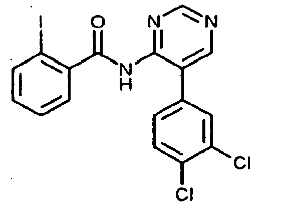 | 500 | 100 |
| (10) | 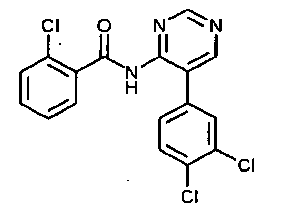 | 500 | 90 |
| (11) | 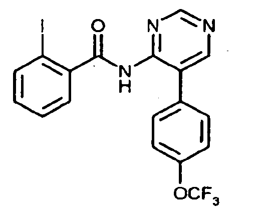 | 500 | 95 |
| (19) | 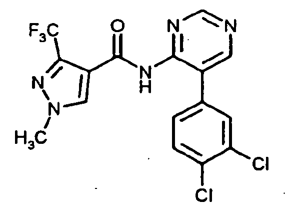 | 500 | 95 |

### Beispiel B

### Alternaria - Test (Tomate) / protektiv

| | | |
|---|---|---|
| Lösungsmittel: | 49 | Gewichtsteile N,N-Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Tomatenpflanzen mit der Wirkstoffzubereitung in der angegebenen Aufwandmenge. 1 Tag nach der Behandlung werden die Pflanzen mit einer Sporensuspension von Alternaria solani inokuliert und stehen dann 24h bei 100 % relativer Feuchte und 20°C. Anschließend stehen die Pflanzen bei 96 % relativer Luftfeuchtigkeit und einer Temperatur von 20°C. 7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle B**

| **Alternaria - Test (Tomate) / protektiv** | | | |
|---|---|---|---|
| Wirkstoff Erfindungsgemäß | | Aufwandmenge an Wirkstoff in ppm | Wirkungsgrad in % |
| (1) | 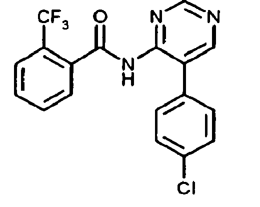 | 500 | 100 |
| (7) | 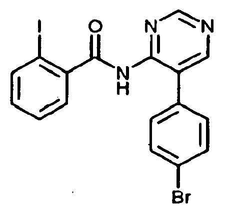 | 500 | 100 |
| (8) | 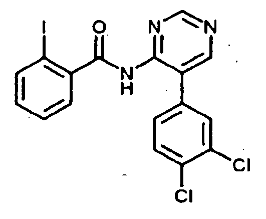 | 500 | 100 |
| (9) | 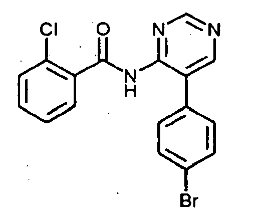 | 500 | 100 |
| (10) | 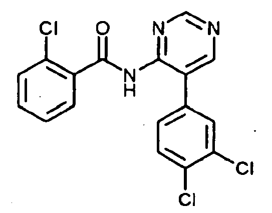 | 500 | 100 |
| (11) | 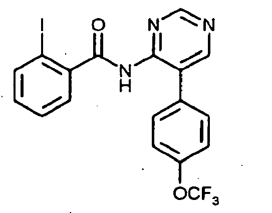 | 500 | 100 |
| (12) | 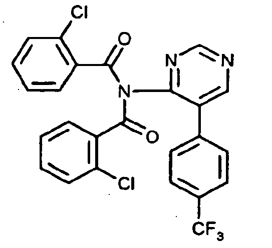 | 500 | 100 |
| (13) | 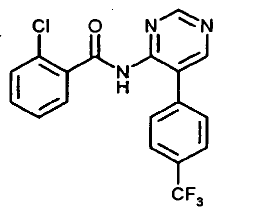 | 500 | 100 |
| (14) | 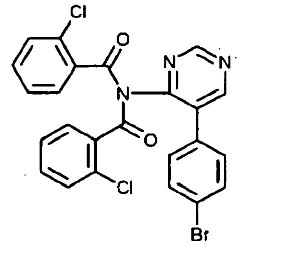 | 500 | 71 |
| (15) | 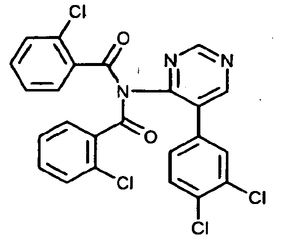 | 500 | 95 |
| (16) | 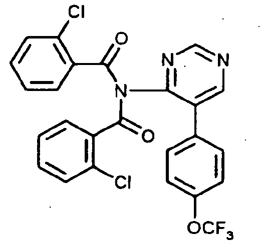 | 500 | 70 |
| (17) | 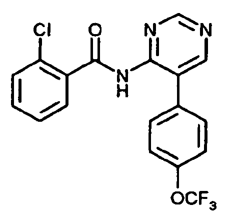 | 500 | 95 |
| (19) | 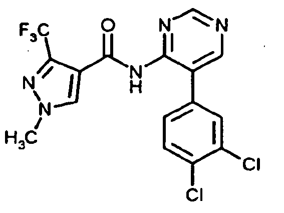 | 500 | 95 |
| (20) | 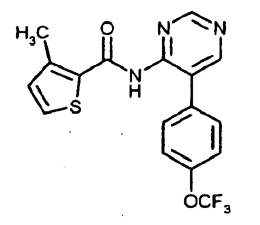 | 500 | 70 |

### Beispiel C

### Pyrenophora teres - Test (Gerste) / protektiv

| | | |
|---|---|---|
| Lösungsmittel: | 50 | Gewichtsteile N,N-Dimethylacetamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.
Zur Prüfung auf protektive Wirksamkeit werden junge Pflanzen mit der Wirkstoffzubereitung in der angegeben Aufwandmenge besprüht. Nach Antrocknen des Spritzbelages werden die Pflanzen mit einer Konidiensuspension von Pyrenophora teres besprüht. Die Pflanzen verbleiben 48 Stunden bei 20°C und 100 % relativer Luftfeuchtigkeit in einer Inkubationskabine. Die Pflanzen werden dann in einem Gewächshaus bei einer Temperatur von ca. 20°C und relativen Luftfeuchtigkeit von ca. 80 % aufgestellt. 8 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle C**

| **Pyrenophora teres - Test (Gerste) / protektiv** | | | |
|---|---|---|---|
| Wirkstoff Erfindungsgemäß | | Aufwandmenge an Wirkstoff in ppm | Wirkungsgrad in % |
| (7) | 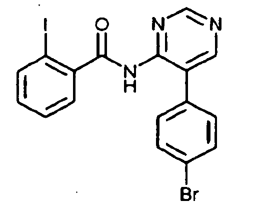 | 1000 | 89 |
| (8) | 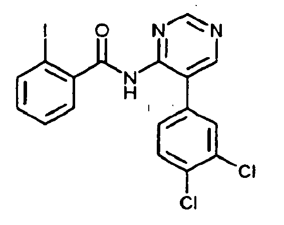 | 1000 | 100 |

### Beispiel D

### Podosphaera - Test (Apfel) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.
Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension des Apfelmehltauerregers *Podosphaera leucotricha* inokuliert. Die Pflanzen werden dann im Gewächshaus bei ca. 23°C und einer relativen Luftfeuchtigkeit von ca. 70 % aufgestellt.
10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle D**

| **Podosphaera - Test (Apfel) / protektiv** | | | |
|---|---|---|---|
| Wirkstoff Erfindungsgemäß | | Aufwandmenge an Wirkstoff in ppm | Wirkungsgrad in % |
| (7) | 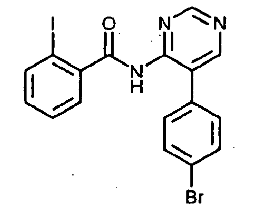 | 100 | 87 |
| (8) | 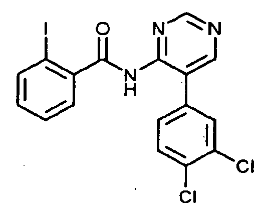 | 100 | 93 |
| (13) | 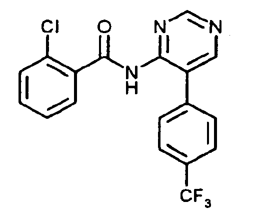 | 100 | 93 |

### Beispiel E

### Venturia - Test (Apfel) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.
Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers *Venturia inaequalis* inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.
10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle E**

| **Venturia - Test (Apfel) / protektiv** | | | |
|---|---|---|---|
| Wirkstoff Erfindungsgemäß | | Aufwandmenge an Wirkstoff in ppm | Wirkungsgrad in % |
| (2) | 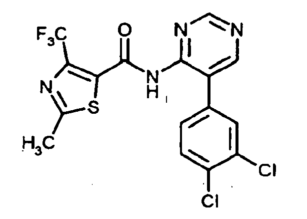 | 100 | 95 |
| (6) | 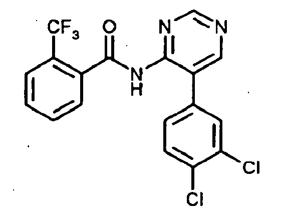 | 100 | 95 |

### Beispiel F

### Botrytis - Test (Bohne) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.
Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit *Botrytis cinerea* bewachsene Agarstückchen aufgelegt. Die inokulierten Pflanzen werden in einer abgedunkelten Kammer bei ca. 20°C und 100 % relativer Luftfeuchtigkeit aufgestellt.

2 Tage nach der Inokulation wird die Größe der Befallsflecken auf den Blättern ausgewertet. Dabei bedeutet 0% ein Wirkungsgrad, der demjenigen der Kontrolle entspricht, während ein Wirkungsgrad von 100 % bedeutet, dass kein Befall beobachtet wird.

**Tabelle F**

| **Botrytis - Test (Bohne) / protektiv** | | | |
|---|---|---|---|
| Wirkstoff Erfindungsgemäß | | Aufwandmenge an Wirkstoff in ppm | Wirkungsgrad in % |
| (2) | 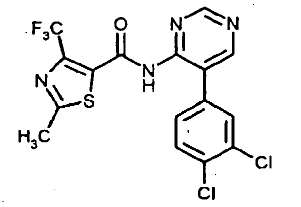 | 500 | 91 |
| (6) | 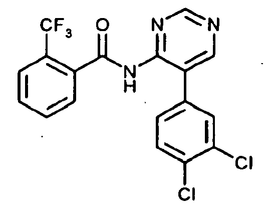 | 500 | 80 |
| (8) | 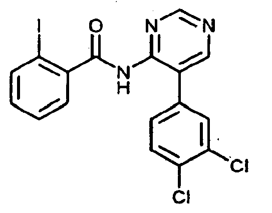 | 500 | 96 |
| (9) | 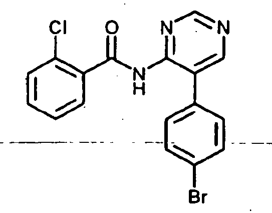 | 500 | 99 |
| (10) | 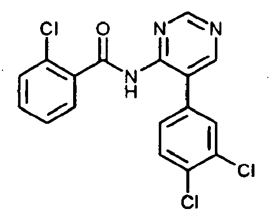 | 500 | 91 |
| (11) | 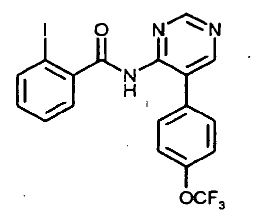 | 500 | 89 |
| (13) | 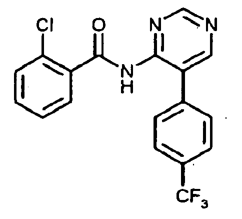 | 500 | 100 |
| (17) | 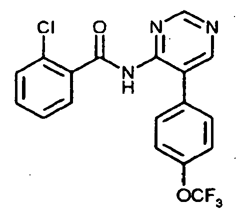 | 500 | 79 |

## Patentansprüche

1. Carboxamide der Formel (I) 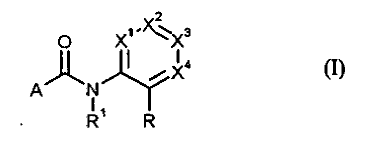 gefunden, in welcher
R für gegebenenfalls einfach bis fünffach durch W¹ substituiertes Phenyl oder die Gruppierung 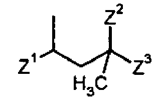 steht,
W¹ für Halogen, Cyano, Nitro, C₁-C₆-Alkyl, C₁-C₄-Alkoxy, C₁-C₄-Alkylthio, C₁-C₄- Alkylsulfonyl, C₂-C₆-Alkenyl, C₃-C₆-Cycloalkyl oder für C₁-C₆-Halogenalkyl, C₁- C₆-Halogenalkoxy, C₁-C₆-Halogenalkylthio oder C₁-C₆-Halogenalkylsulfonyl mit jeweils 1 bis 13 Halogenatomen, oder für -C(Q²)=N-Q³ steht, worin
Q² für Wasserstoff, Hydroxy, C₁-C₄-Alkyl, C₁-C₄-Halogenalkyl 1 bis 9 Halogenatomen oder C₃-C₆-Cycloalkyl steht,
Q³ für Hydroxy, Amino, Methylamino, Phenyl, Benzyl oder für jeweils gegebenenfalls durch Halogen, Cyano, Hydroxy, C₁-C₄-Alkoxy, C₁-C₄-Alkylthio, C₁-C₄-Alkyl- amino, Di(C₁-C₄-alkyl)amino oder Phenyl substituiertes C₁-C₄-Alkyl oder C₁-C₄- Alkoxy, oder für C₂-C₄-Alkenyloxy oder C₂-C₄-Alkinyloxy steht,
Z¹ für Wasserstoff oder Methyl steht,
Z² für Wasserstoff oder Methyl steht,
Z³ für Methyl oder Ethyl steht,
R¹ für Wasserstoff, C₁-C₈-Alkyl, C₁-C₆-Alkylsulfinyl, C₁-C₆-Alkylsulfonyl, C₁-C₄- Alkoxy-C₁-C₄-alkyl, C₃-C₈-Cycloalkyl; C₁-C₆-Halogenalkyl, C₁-C₄-Halogenalkyl- thio, C₁-C₄-Halogenalkylsulfinyl, C₁-C₄-Halogenalkylsulfonyl, Halogen-C₁-C₄- alkoxy-C₁-C₄-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃- alkyl, (C₁-C₃-Alkoxy)carbonyl-C₁₋C₃-alkyl; Halogen-(C₁-C₃-alkyl)carbonyl-C₁-C₃- alkyl, Halogen-(C₁-C₃-alkoxy)carbonyl-C₁-C₃-alkyl mit jeweils 1 bis 13 Fluor-, Chlor- und/oder Bromatomen;
(C₁-C₈-Alkyl)carbonyl, (C₁-C₈-Alkoxy)carbonyl, (C₁-C₈-Alkylthio)carbonyl, (C₁-C₄- Alkoxy-C₁-C₄-alkyl)carbonyl, (C₃-C₆-Alkenyloxy)carbonyl, (C₃-C₆-Alkinyloxy)car- bonyl, (C₃-C₈-Cycloalkyl)carbonyl; (C₁-C₆-Halogenalkyl)carbonyl, (C₁-C₆-Halogen- alkoxy)carbonyl, (C₁-C₆-Halogenalkylthio)carbonyl, (Halogen-C₂-C₄-alkoxy-C₁-C₄- alkyl)carbonyl, (C₃-C₆-Halogenalkenyloxy)carbonyl, (C₃-C₆-Halogenalkinyloxy)car- bonyl, (C₃-C₈-Halogencycloalkyl)carbonyl mit jeweils 1 bis 9 Fluor-, Chlor- und/oder Bromatomen; oder -CH₂-C≡C-R^{1-A}, -CH₂-CH=CH-R^{1-A}, -CH=C=CH-R^{1-A}, -C(=O)C(=O)R², -CONR³R⁴ oder -CH₂NR⁵R⁶ steht,
R^{1-A} für Wasserstoff, C₁-C₆-Alkyl, C₁-C₆-Halogenalkyl, C₂-C₆-Alkenyl, C₂-C₆-Alkinyl, C₃-C₇-Cycloalkyl, (C₁-C₄-Alkoxy)carbonyl, (C₃-C₆-Alkenyloxy)carbonyl, (C₃-C₆- Alkinyloxy)carbonyl oder Cyano steht,
R² für Wasserstoff, C₁-C₈-Alkyl, C₁-C₈-Alkoxy, C₁-C₄-Alkoxy-C₁-C₄-alkyl C₃-C₈- Cycloalkyl; C₁-C₆-Halogenalkyl, C₁-C₆-Halogenalkoxy, Halogen-C₁-C₄-alkoxy-C₁- C₄-alkyl, C₃-C₈-Halogencycloalkyl mit jeweils 1 bis 9 Fluor-, Chlor- und/oder Bromatomen steht,
R³ und R^{a} unabhängig voneinander jeweils für Wasserstoff, C₁-C₈-Alkyl, C₁-C₄-Alkoxy-C₁- C₄-alkyl, C₃-C₈-Cycloalkyl, C₁-C₈-Halogenalkyl, Halogen-C₁-C₄-alkoxy-C₁-C₄- alkyl, C₃-C₈-Halogencycloalkyl mit jeweils 1 bis 9 Fluor-, Chlor- und/oder Bromatomen stehen,
R³ und R⁴ außerdem gemeinsam mit dem Stickstoffatom, an das sie gebunden sind, einen ge- gebenenfalls einfach oder mehrfach, gleich oder verschieden durch Halogen oder C₁- C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR⁷ enthalten kann,
R⁵ und R⁶ unabhängig voneinander für Wasserstoff, C₁-C₈-Alkyl, C₃-C₈-Cycloalkyl; C₁-C₈- Halogenalkyl, C₃-C₈-Halogencycloalkyl mit jeweils 1 bis 9 Fluor-, Chlor- und/oder Bromatomen stehen,
R⁵ und R⁶ außerdem gemeinsam mit dem Stickstoffatom, an das sie gebunden sind, einen ge- gebenenfalls einfach oder mehrfach, gleich oder verschieden durch Halogen oder C₁- C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR⁷ enthalten kann,
R⁷ für Wasserstoff oder C₁-C₆-Alkyl steht,
X¹, X², X³ und X⁴ unabhängig voneinander für N oder CR⁸ stehen mit der Maßgabe, dass wenigstens einer dieser Reste für N steht,
R⁸ für Wasserstoff, Fluor, Chlor, Methyl, iso-Propyl, Methylthio oder Trifluormethyl steht,
A für einen der folgenden Reste A1 bis A18 steht 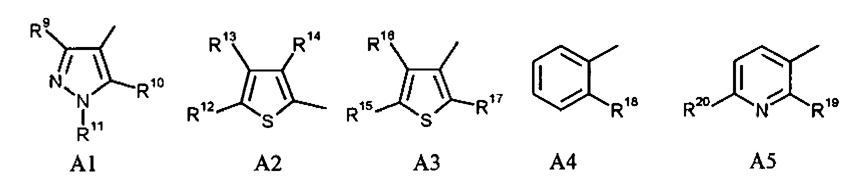 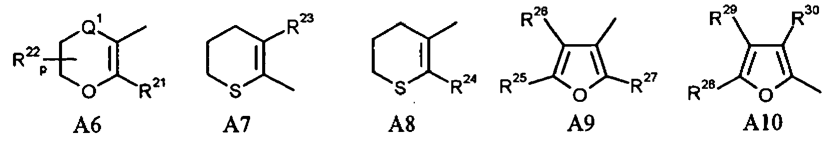 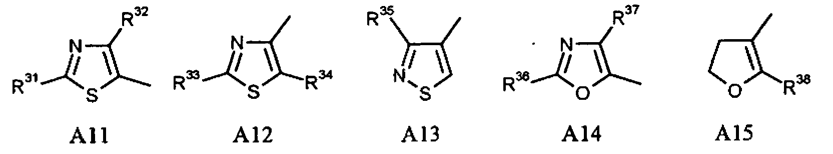 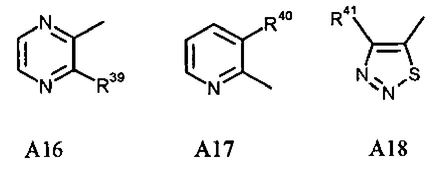
R⁹ für Wasserstoff, Cyano, Halogen, Nitro, C₁-C₄-Alkyl, C₁-C₄-Alkoxy, C₁-C₄-Alkyl- thio, C₃-C₆-Cycloalkyl, C₁-C₄-Halogenalkyl, C₁-C₄-Halogenalkoxy oder C₁-C₄-Halo- genalkylthio mit jeweils 1 bis 5 Halogenatomen, Aminocarbonyl oder Aminocar- bonyl-C₁-C₄-alkyl steht,
R¹⁰ für Wasserstoff, Halogen, Cyano, C₁-C₄-Alkyl, C₁-C₄-Alkoxy oder C₁-C₄-Alkylthio steht,
R¹¹ für Wasserstoff, C₁-C₄-Alkyl, Hydroxy-C₁-C₄-alkyl, C₂-C₆-Alkenyl, C₃-C₆-Cycloalkyl, C₁-C₄-Alkylthio-C₁-C₄-alkyl, C₁-C₄-Alkoxy-C₁-C₄-alkyl, C₁-C₄-Halogenalkyl, C₁-C₄- Halogenalkylthio-C₁-C₄-alkyl, C₁-C₄-Halogenalkoxy-C₁-C₄-alkyl mit Jeweils 1 bis 5 Halogenatomen, Phenyl, (C₁-C₄-Alkyl)carbonyl, (C₁-C₄-Alkoxy)carbonyl, (C₁-C₄- Atkylthio)carbonyl, (C₁-C₄-Alkoxy-C₁-C₄-alkyl)carbonyl; (C₁-C₄-Halogenalkyl)car- bonyl, (C₁-C₄-Halogenalkoxy)carbonyl, (C₁-C₄-Halogenalkylthio)carbonyl, (Ha- logen-C₁-C₄-alkoxy-C₁-C₄-alkyl)carbonyl mit jeweils 1 bis 9 Halogenatomen steht,
R¹² und R¹³ unabhängig voneinander für Wasserstoff, Halogen, C₁-C₄-Alkyl oder C₁-C₄- Halogenalkyl mit 1 bis 5 Halogenatomen steht,
R¹⁴ für Halogen, Cyano oder C₁-C₄-Alkyl, oder C₁-C₄-Hogenalkyl oder C₁-C₄-Halo- genalkoxy mit jeweils 1 bis 5 Halogenatomen steht,
R¹⁵ und R¹⁶ unabhängig voneinander für Wasserstoff, Halogen, C₁-C₄-Alkyl oder C₁-C₄- Halogenalkyl mit 1 bis 5 Halogenatomen steht,
R¹⁷ für Wasserstoff, C₁-C₄-Alkyl oder C₁-C₄-Halogenalkyl mit 1 bis 5 Halogenatomen steht,
R¹⁸ für Wasserstoff Halogen, Hydroxy, Cyano, C₁-C₆-Alkyl, C₁-C₄-Halognalkyl, C₁-C₄- Halogenalkoxy oder C₁-C₄-Halogenalkylthio mit jeweils 1 bis 5 Halogenatomen steht,
R¹⁹ für Halogen, Hydroxy, Cyano, C₁-C₄-Alkyl, C₁-C₄-Alkoxy, C₁-C₄-Alkylthio, C₁-C₄- Halogenalkyl, C₁-C₄-Halogenalkylthio oder C₁-C₄-Halogenalkoxy mit jeweils 1 bis 5 Halogenatomen steht,
R²⁰ für Wasserstoff, Halogen, Cyano, C₁-C₄-Alkyl, C₁-C₄-Alkoxy, C₁-C₄-Alkylthio, C₁- C₄-Halogenalkyl, C₁-C₄-Halogenalkoxy mit jeweils 1 bis 5 Halogenatomen, C₁-C₄- Alkylsulphinyl oder C₁-C₄-Alkylsulphonyl steht,
R²¹ für C₁-C₄-Alkyl oder C₁-C₄-Halogenalkyl mit 1 bis 5 Halogenatomen steht,
R²² für C₁-C₄-Alkyl steht,
Q¹ für S (Schwefel), SO, SO₂ oder CH₂ steht,
p für 0, 1 oder 2, wobei R²² für identische oder verschiedene Reste steht, wenn p für 2 steht,
R²³ für C₁-C₄-Alkyl oder C₁-C₄-Halogenalkyl mit 1 bis 5 Halogenatomen steht,
R²⁴ für C₁-C₄-Alkyl oder C₁-C₄-Halogenalkyl mit 1 bis 5 Halogenatomen steht,
R²⁵ und R²⁶ unabhängig voneinander für Wasserstoff, Halogen, Amino, C₁-C₄-Alkyl oder C₁-C₄-Halogenalkyl mit 1 bis 5 Halogenatomen stehen,
R²⁷ für Wasserstoff, C₁-C₄-Alkyl oder C₁-C₄-Halogenalkyl mit 1 bis 5 Halogenatomen steht,
R²⁸ und R²⁹ unabhängig voneinander für Wasserstoff, Halogen, Amino, Nitro, C₁-C₄-Alkyl oder C₁-C₄-Halogenalkyl mit 1 bis 5 Halogenatomen stehen,
R³⁰ für Wasserstoff, Halogen, C₁-C₄-Alkyl oder C₁-C₄-Halogenalkyl mit 1 bis 5 Halogenatomen steht,
R³¹ für Wasserstoff, Halogen, Amino, C₁-C₄-Alkylamino, Di-(C₁-C₄-alkyl)amino, Cyano, C₁-C₄-Alkyl oder C₁-C₄-Halogenalkyl mit 1 bis 5 Halogenatomen steht,
R³² für Halogen, C₁-C₄-Alkyl oder C₁-C₄-Halogenalkyl mit 1 bis 5 Halogenatomen steht,
R³³ für Wasserstoff, Halogen, Amino, C₁-C₄-Aklamino, Di-(C₁-C₄-alkyl)amino, Cyano, C₁-C₄-Alkyl oder C₁-C₄-Halogenalkyl mit 1 bis 5 Halogenatomen steht,
R³⁴ für Halogen, C₁-C₄-Alkyl oder C₁-C₄-Halogenalkyl mit 1 bis 5 Halogenatomen steht,
R³⁵ für Halogen, C₁-C₄-Alkyl oder C₁-C₄-Halogenalkyl mit 1 bis 5 Halogenatomen steht,
R³⁶ für Wasserstoff oder C₁-C₄-Alkyl steht,
R³⁷ für Halogen oder C₁-C₄-Alkyl steht,
R³⁸ für C₁-C₄-Alkyl oder C₁-C₄-Halogenalkyl mit 1 bis 5 Halogenatomen steht,
R³⁹ für Wasserstoff, Halogen, C₁-C₄₋Alkyl oder C₁-C₄-Halogenalkyl mit 1 bis 5 Halo- genatomen steht,
R⁴⁰ für Halogen, Hydroxy, C₁-C₄-Alkyl, C₁-C₄-Alkoxy, C₁-C₄-Alkylthio, C₁-C₄-Halo- genalkyl, C₁-C₄-Halogenalkylthio oder C₁-C₄-Halogenalkoxy mit jeweils 1 bis 5 Halogenatomen steht,
R⁴¹ für C₁-C₄-Alkyl steht.

2. Verfahren zum Herstellen von Carboxamiden der Formel (1) gemäß Anspruch 1, **dadurch gekennzeichnet, dass** man
(a) Carbonsäurehalogenide der Formel (II) 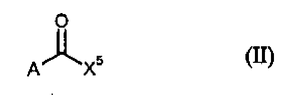 in welcher
A die in Anspruch 1 angegebenen Bedeutungen hat,
X⁵ für Halogen oder Hydroxy steht, mit Anilinderivaten der Formel (III) 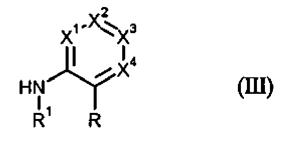 in welcher
R, R¹, X¹, X², X³ und X⁴ die in Anspruch 1 angegebenen Bedeutungen haben, gegebenenfalls in Gegenwart eines Kupplungsreagenzes, gegebenenfalls in Gegenwart eines Säurebindemittels und gegebenenfalls in Gegenwart eines Verdünnungsmittels umsetzt,
oder
(b) Carboxamide der Formel (I-a) 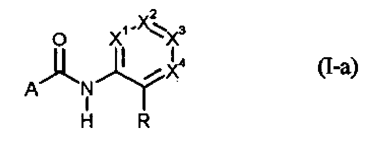 in welcher
R, X¹, X², X³, X⁴ und A die in Anspruch 1 angegebenen Bedeutungen haben, mit Halogeniden der Formel (TV)
R^{1-B}-Hal (IV)
in welcher
R^{1-B} für C₁-C₈-Alkyl, C₁-C₆-Alkylsulfinyl, C₁-C₆-Alkylsulfonyl, C₁-C₄-Alkoxy- C₁-C₄-alkyl, C₃-C₈-Cycloalkyl; C₁-C₆-Halogenalkyl, C₁-C₄-Halogenalkyl- thio, C₁-C₄-Halogenalkylsulfinyl, C₁-C₄-Halogenalkylsulfonyl, Halogen-C₁- C₄-alkoxy-C₁-C₄-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)- carbonyl-C₁-C₈-alkyl, (C₁-C₃-Alkoxy)carbonyl-C₁-C₃-alkyl; Halogen-(C₁- C₃-alkyl)carbonyl-C₁-C₃-alkyl, Halogen-(C₁-C₃-alkoxy)carbonyl-C₁-C₃- alkyl mit jeweils 1 bis 13 Fluor-, Chlor-und/oder Bromatomen; (C₁-C₈-Alkyl)carbonyl, (C₁-C₈-Alkoxy)carbonyl, (C₁-C₈-Alkylthio)carbonyl, (C₁-C₄-Alkoxy-C₁-C₄-alkyl)carbonyl, (C₃-C₆-Alkenyloxy)carbonyl, (C₃-C₆- Alkinyloxy)carbonyl, (C₃-C₈-Cycloalkyl)carbonyl; (C₁-C₆-Halogenalkyl)- carbonyl, (C₁-C₆-Halogenalkoxy)carbonyl, (C₁-C₆-Halogenalkylthio)car- bonyl, (Halogen-C₁-C₄-alkoxy-C₁-C₄-alkyl)carbonyl, (C₃-C₆-Halogen- alkenyloxy)carbonyl, (C₃-C₆-Halogenalkinyloxy)carbonyl, (C₃-C₈-Halogen- cycloalkyl)carbonyl mit jeweils 1 bis 9 Fluor-, Chlor- und/oder Brom- atomen; oder -CH₂-C=C-R^{1-A}, -CH₂-CH=CH-R^{1-A}, -CH=C=CH-R^{1-A}, -C(=O)C(=O)R², -CONR³R⁴ oder -CH₂NR⁵R⁶ steht,
R^{1-A}, R², R³, R⁴, R⁵ und R⁶ die in Anspruch 1 angegebenen Bedeutungen haben,
Hal für Chlor, Brom oder Iod steht,
in Gegenwart einer Base und in Gegenwart eines Verdünnungsmittels umsetzt.

3. Mittel zum Bekämpfen unerwünschter Mikroorganismen, **gekennzeichnet durch** einen Gehalt an mindestens einem Carboxamid der Formel (I) gemäß Anspruch 1 neben Streckmitteln und/oder oberflächenaktiven Stoffen.

4. Verwendung von Carboxamiden der Formel (I) gemäß Anspruch 1 zum Bekämpfen unerwünschter Mikroorganismen.

5. Verfahren zum Bekämpfen unerwünschter Mikroorganismen, **dadurch gekennzeichnet, dass** man Carboxamide der Formel (I) gemäß Anspruch 1 auf die Mikroorganismen und/oder deren Lebensraum ausbringt.

6. Verfahren zum Herstellen von Mitteln zum Bekämpfen unerwünschter Mikroorganismen, **dadurch gekennzeichnet, dass** man Carboxamide der Formel (I) gemäß Anspruch 1 mit Streckmitteln und/oder oberflächenaktiven Stoffen vermischt.

7. Verwendung von Carboxamiden der Formel (I) gemäß Anspruch 1 zur Behandlung von Saatgut.

8. Verwendung von Carboxamiden der Formel (I) gemäß Anspruch 1 zur Behandlung von transgenen Pflanzen.

9. Verwendung von Carboxamiden der Formel (I) gemäß Anspruch 1 zur Behandlung von Saatgut transgener Pflanzen.

10. Anilinderivates der Formel (III-a) 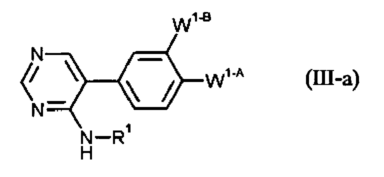 in welcher
R¹ die in Anspruch 1 angegebenen Bedeutungen hat,
und
W^{1-A} und W^{1-B} entweder gleichzeitig für Chlor stehen
oder
W^{1-A} für Trifluormethyl oder Trifluormethoxy steht und W^{1-B} für Wasserstoff steht.

## Claims

1. Carboxamides of the formula (I) 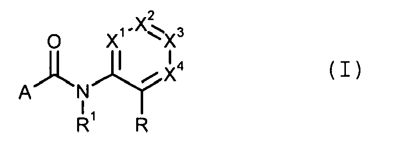 in which
R represents phenyl which is optionally mono- to pentasubstituted by W¹ or represents the grouping 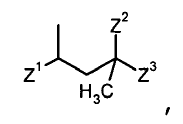
W¹ represents halogen, cyano, nitro, C₁-C₆-alkyl, C₁-C₄-alkoxy, C₁-C₄-alkylthio, C₁-C₄- alkylsulphonyl, C₂-C₆-alkenyl, C₃-C₆-cycloalkyl or represents C₁-C₆-haloalkyl, C₁-C₆- haloalkoxy, C₁-C₆-haloalkylthio or C₁-C₆- haloalkylsulphonyl having in each case 1 to 13 halogen atoms, or represents -C(Q²)=N-Q³ in which
Q² represents hydrogen, hydroxyl, C₁-C₄-alkyl, C₁- C₄-haloalkyl having 1 to 9 halogen atoms or C₃- C₆-cycloalkyl,
Q³ represents hydroxyl, amino, methylamino, phenyl, benzyl or represents in each case optionally halogen-, cyano-, hydroxyl-, C₁-C₄- alkoxy-, C₁-C₄-alkylthio-, C₁-C₄-alkyamino, di(C₁-C₄-alkyl)amino or phenyl-substituted C₁- C₄-alkyl or C₁-C₉-alkoxy, or represents C₂-C₄- alkenyloxy or C₂-C₄-Alkynyloxy,
Z¹ represents hydrogen or methyl,
Z² represents hydrogen or methyl,
Z³ represents methyl or ethyl,
R¹ represents hydrogen, C₁-C₈-alkyl, C₁-C₆- alkylsulphinyl, C₁-C₆-alkylsulphonyl, C₁-C₄- alkoxy-C₁-C₄-alkyl, C₃-C₈-cycloalkyl; C₁-C₆- haloalkyl, C₁-C₄-haloalkylthio, C₁-C₄- haloalkylsulphinyl, C₁-C₄-haloalkylsulphonyl, halo-C₁-C₄-alkoxy-C₁-C₄-alkyl, C₃-C₈- halocycloalkyl having in each case 1 to 9 fluorine, chlorine and/or bromine atoms; formyl, formyl-C₁-C₃-alkyl, (C₁-C₃- alkyl) carbonyl-C₁-C₃-alkyl, (C₁-C₃-alkoxy)- carbonyl-C₁-C₃-alkyl; halo- (C₁-C₃- alkyl) carbonyl-C₁-C₃-alkyl, halo- (C₁-C₃- alkoxy)carbonyl-C₁-C₃-alkyl having in each case 1 to 13 fluorine, chlorine and/or bromine atoms;
(C₁-C₈-alkyl)carbonyl, (C₁-C₈-alkoxy)carbonyl, (C₁-C₈-alkylthio)carbonyl, (C₁-C₄-alkoxy-C₁-C₄- alkyl)carbonyl, (C₃-C₆-alkenyloxy)carbonyl, (C₃-C₆-alkynyloxy) carbonyl, (C₃-C₈- cycloalkyl)carbonyl; (C₁-C₆-haloalkyl)carbonyl, (C₁-C₆-haloalkoxy) carbonyl, (C₁-C₆- haloalkylthio)carbonyl, (halo-C₁-C₄-alkoxy- C₁-C₄-alkyl)carbonyl, (C₃-C₆- haloalkenyloxy)carbonyl, (C₃-C₆- haloalkynyloxy)carbonyl, (C₃-C₈- halocycloalkyl)carbonyl having in each case 1 to 9 fluorine, chlorine and/or bromine atoms; or -CH₂-C≡ C-R^{1-A}, -CH₂-CH=CH-R^{1-A}, -CH=C=CH-R^{1-A}, -C(=O)C(=O)R², -CONR³R⁴ or -CH₂NR⁵R⁶,
R^{1-A} represents hydrogen, C₁-C₆-alkyl, C₁-C₆- haloalkyl, C₂-C₆-alkenyl, C₂-C₆-alkynyl, C₃-C₇- cycloalkyl, (C₁-C₄-alkoxy)carbonyl, (C₃-C₆- alkenyloxy) carbonyl, (C₃-C₆-alkynyloxy) carbonyl or cyano,
R² represents hydrogen, C₁-C₈-alkyl, C₁-C₈-alkoxy, C₁-C₄-alkoxy-C₁-C₄-alkyl, C₃-C₈-cycloalkyl; C₁- C₆-haloalkyl, C₁-C₆-haloalkoxy, halo-C₁-C₄- alkoxy-C₁-C₄-alkyl, C₃-C₈-halocycloalkyl having in each case 1 to 9 fluorine, chlorine and/or bromine atoms,
R³ and R⁴ independently of one another each represent hydrogen, C₁-C₈-alkyl, C₁-C₄-alkoxy- C₁-C₄-alkyl, C₃-C₈-cycloalkyl; C₁-C₈-haloalkyl, halo-C₁-C₄-alkoxy-C₁-C₄-alkyl, C₃-C₈- halocycloalkyl having in each case 1 to 9 fluorine, chlorine and/or bromine atoms,
R³ and R⁴ furthermore together with the nitrogen atom to which they are attached form a saturated heterocycle which is optionally mono- or polysubstituted by identical or different substituents from a group consisting of halogen and C₁-C₄-alkyl and which has 5 to 8 ring atoms, where the heterocycle may contain 1 or 2 further non-adjacent heteroatoms from the group consisting of oxygen, sulphur and NR⁷,
R⁵ and R⁶ independently of one another represent hydrogen, C₁-C₈-alkyl, C₃-C₈-cycloalkyl; C₁-C₈- haloalkyl, C₃-C₈-halocycloalkyl having in each case 1 to 9 fluorine, chlorine and/or bromine atoms,
R⁵ and R⁶ furthermore together with the nitrogen atom to which they are attached form a saturated heterocycle which is optionally mono- or polysubstituted by identical or different substituents from a group consisting of halogen and C₁-C₄-alkyl and which has 5 to 8 ring atoms, where the heterocycle may contain 1 or 2 further non-adjacent heteroatoms from the group consisting of oxygen, sulphur and NR⁷,
R⁷ represents hydrogen or C₁-C₆-alkyl,
X¹, X², X³ and X⁴ independently of one another represent N or CR⁸, with the proviso that at least one of these radicals represents N,
R⁸ represents hydrogen, fluorine, chlorine, methyl, isopropyl, methylthio or trifluoromethyl,
A represents one of the radicals A1 to A18 below 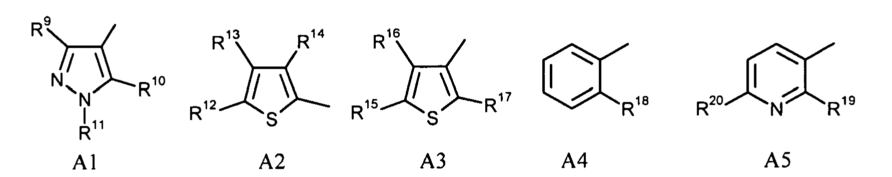 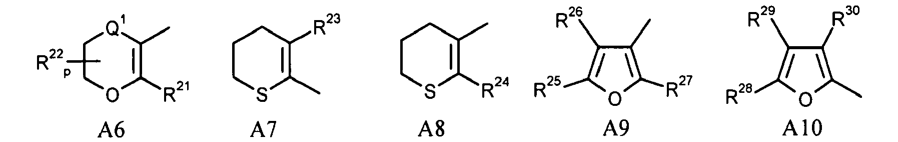 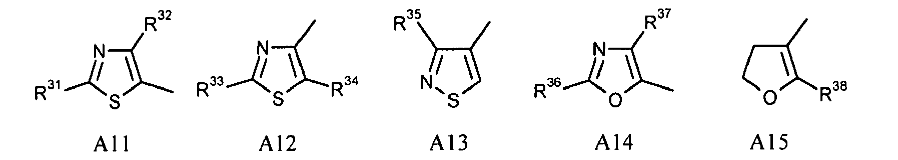 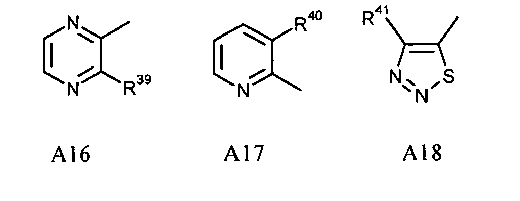
R⁹ represents hydrogen, cyano, halogen, nitro, C₁-C₄-alkyl, C₁-C₄-alkoxy, C₁-C₄-alkylthio, C₃- C₆-cycloalkyl, C₁-C₄-haloalkyl, C₁-C₄-haloalkoxy or C₁-C₄-haloalkylthio having in each case 1 to 5 halogen atoms, aminocarbonyl or aminocarbonyl-C₁-C₄-alkyl,
R¹⁰ represents hydrogen, halogen, cyano, C₁-C₄- alkyl, C₁-C₄-alkoxy or C₁-C₄-alkylthio,
R¹¹ represents hydrogen, C₁-C₄-alkyl, hydroxy-C₁-C₄- alkyl, C₂-C₆-alkenyl, C₃-C₆-cycloalkyl, C₁-C₄- alkylthio-C₁-C₄-alkyl, C₁-C₄-alkoxy-C₁-C₄-alkyl, C₁-C₄-haloalkyl, C₁-C₄-haloalkylthio-C₁-C₄-alkyl, C₁-C₄-haloalkoxy-C₁-C₄-alkyl having in each case 1 to 5 halogen atoms, phenyl, (C₁-C₄- alkyl) carbonyl, (C₁-C₄-alkoxy)carbonyl, (C₁-C₄- alkylthio) carbonyl, (C₁-C₄-alkoxy-C₁-C₄- alkyl) carbonyl; (C₁-C₄-haloalkyl)carbonyl, (C₁- C₄-haloalkoxy)carbonyl, (C₁-C₄- haloalkylthio)carbonyl, (halo-C₁-C₄-alkoxy- C₁-C₄-alkyl)carbonyl having in each case 1 to 9 halogen atoms,
R¹² and R¹³ independently of one another represent hydrogen, halogen, C₁-C₄-alkyl or C₁-C₄- haloalkyl having 1 to 5 halogen atoms,
R¹⁴ represents halogen, cyano or C₁-C₄-alkyl, or C₁-C₄-haloalkyl or C₁-C₄-haloalkoxy having in each case 1 to 5 halogen atoms,
R¹⁵ and R¹⁶ independently of one another represent hydrogen, halogen, C₁-C₄-alkyl or C₁-C₄- haloalkyl having 1 to 5 halogen atoms,
R¹⁷ represents hydrogen, C₁-C₄-alkyl or C₁-C₄- haloalkyl having 1 to 5 halogen atoms,
R¹⁸ represents hydrogen, halogen, hydroxyl, cyano, C₁-C₆-alkyl, C₁-C₄-haloalkyl, C₁-C₄-haloalkoxy or C₁-C₄-haloalkylthio having in each case 1 to 5 halogen atoms,
R¹⁹ represents halogen, hydroxyl, cyano, C₁-C₄- alkyl, C₁-C₄-alkoxy, C₁-C₄-alkylthio, C₁-C₄- haloalkyl, C₁-C₄-haloalkylthio or C₁-C₄- haloalkoxy having in each case 1 to 5 halogen atoms,
R²⁰ represents hydrogen, halogen, cyano, C₁-C₄- alkyl, C₁-C₄-alkoxy, C₁-C₄-alkylthio, C₁-C₄- haloalkyl, C₁-C₄-haloalkoxy having in each case 1 to 5 halogen atoms, C₁-C₄-alkylsulphinyl or C₁-C₄-alkylsulphonyl,
R²¹ represents C₁-C₄-alkyl or C₁-C₄-haloalkyl having 1 to 5 halogen atoms,
R²² represents C₁-C₄-alkyl,
Q¹ represents S (sulphur), SO, SO₂ or CH₂,
p represents 0, 1 or 2, where R²² represents identical or different radicals if p represents 2,
R²³ represents C₁-C₄-alkyl or C₁-C₄-haloalkyl having 1 to 5 halogen atoms,
R²⁴ represents C₁-C₄-alkyl or C₁-C₄-haloalkyl having 1 to 5 halogen atoms,
R²⁵ and R²⁶ independently of one another represent hydrogen, halogen, amino, C₁-C₄-alkyl or C₁-C₄- haloalkyl having 1 to 5 halogen atoms,
R²⁷ represents hydrogen, C₁-C₄-alkyl or C₁-C₄- haloalkyl having 1 to 5 halogen atoms,
R²⁸ and R²⁹ independently of one another represent hydrogen, halogen, amino, nitro, C₁-C₄-alkyl or C₁-C₄-haloalkyl having 1 to 5 halogen atoms,
R³⁰ represents hydrogen, halogen, C₁-C₄-alkyl or C₁-C₄-haloalkyl having 1 to 5 halogen atoms,
R³¹ represents hydrogen, halogen, amino, C₁-C₄- alkylamino, di-(C₁-C₄-alkyl)amino, cyano, C₁- C₄-alkyl or C₁-C₄-haloalkyl having 1 to 5 halogen atoms,
R³² represents halogen, C₁-C₄-alkyl or C₁-C₄- haloalkyl having 1 to 5 halogen atoms,
R³³ represents hydrogen, halogen, amino, C₁-C₄- alkylamino, di-(C₁-C₄-alkyl)amino, cyano, C₁- C₄-alkyl or C₁-C₄-haloalkyl having 1 to 5 halogen atoms,
R³⁹ represents halogen, C₁-C₄-alkyl or C₁-C₄- haloalkyl having 1 to 5 halogen atoms,
R³⁵ represents halogen, C₁-C₄-alkyl or C₁-C₄- haloalkyl having 1 to 5 halogen atoms,
R³⁶ represents hydrogen or C₁-C₄-alkyl,
R³⁷ represents halogen or C₁-C₄-alkyl,
R³⁸ represents C₁-C₄-alkyl or C₁-C₄-haloalkyl having 1 to 5 halogen atoms,
R³⁹ represents hydrogen, halogen, C₁-C₄-alkyl or C₁-C₄-haloalkyl having 1 to 5 halogen atoms,
R⁴⁰ represents halogen, hydroxyl, C₁-C₄-alkyl, C₁- C₄-alkoxy, C₁-C₄-alkylthio, C₁-C₄-haloalkyl, C₁- C₄-haloalkylthio or C₁-C₄-haloalkoxy having in each case 1 to 5 halogen atoms,
R⁴¹ represents C₁-C₄-alkyl.

2. Process for preparing carboxamides of the formula (I) according to Claim 1, **characterized in that**
(a) carbonyl halides of the formula (II) 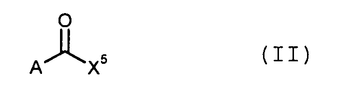 in which
A is as defined in Claim 1,
X⁵ represents halogen or hydroxyl,
are reacted with aniline derivatives of the formula (III) 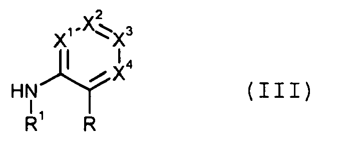 in which
R, R¹, X¹, X², X³ and X⁴ are as defined in Claim 1,
if appropriate in the presence of a coupling agent, if appropriate in the presence of an acid binder and if appropriate in the presence of a diluent,
or
(b) carboxamides of the formula (I-a) 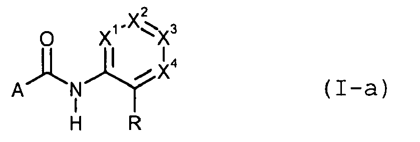 in which
R, X¹, X², X³, X⁴ and A are as defined in Claim 1
are reacted with halides of the formula (IV)
R^{1-B}―Hal (IV)
in which
R^{1-B} represents C₁-C₈-alkyl, C₁-C₆- alkylsulphinyl, C₁-C₆-alkylsulphonyl, C₁-C₄-alkoxy-C₁-C₄-alkyl, C₃-C₈-cycloalkyl; C₁-C₆-haloalkyl, C₁-C₄-haloalkylthio, C₁- C₄-haloalkylsulphinyl, C₁-C₄- haloalkylsulphonyl, halo-C₁-C₄-alkoxy- C₁-C₄-alkyl, C₃-C₈-halocycloalkyl having in each case 1 to 9 fluorine, chlorine and/or bromine atoms; formyl, formyl-C₁- C₃-alkyl, (C₁-C₃-alkyl)carbonyl-C₁-C₃- alkyl, (C₁-C₃-alkoxy) carbonyl-C₁-C₃-alkyl; halo (C₁-C₃-alkyl) carbonyl-C₁-C₃-alkyl, halo (C₁-C₃-alkoxy) carbonyl-C₁-C₃-alkyl having in each case 1 to 13 fluorine, chlorine and/or bromine atoms;
(C₁-C₈-alkyl)carbonyl, (C₁-C₈- alkoxy)carbonyl, (C₁-C₈- alkylthio)carbonyl, (C₁-C₄-alkoxy-C₁-C₄- alkyl)carbonyl, (C₃-C₆- alkenyloxy)carbonyl, (C₃-C₆- alkynyloxy)carbonyl, (C₃-C₈- cycloalkyl)carbonyl; (C₁-C₆- haloalkyl)carbonyl, (C₁-C₆- haloalkoxy)carbonyl, (C₁-C₆- haloalkylthio)carbonyl, (halo-C₁-C₄- alkoxy-C₁-C₄-alkyl)carbonyl, (C₃-C₆- haloalkenyloxy)carbonyl, (C₃-C₆- haloalkynyloxy)carbonyl, (C₃-C₈- halocycloalkyl)carbonyl having in each case 1 to 9 fluorine, chlorine and/or bromine atoms; or -CH₂-C≡ C-R^{1-A}, -CH₂-CH=CH-R^{1-A}, -CH=C=CH-R^{1-A}, -C(=O)C(=O)R², -CONR³R⁴ or -CH₂NR⁵R⁶,
R^{1-A}, R², R³, R⁴, R⁵ and R⁶ are as defined in Claim 1,
Hal represents chlorine, bromine or iodine,
in the presence of a base and in the presence of a diluent.

3. Compositions for controlling unwanted microorganisms, **characterized in that** they comprise at least one carboxamide of the formula (I) according to Claim 1, in addition to extenders and/or surfactants.

4. Use of carboxamides of the formula (I) according to Claim 1 for controlling unwanted microorganisms in crop protection and in the protection of materials.

5. Method for controlling unwanted microorganisms in crop protection and in the protection of materials, **characterized in that** carboxamides of the formula (I) according to Claim 1 are applied to the microorganisms and/or their habitat.

6. Process for preparing compositions for controlling unwanted microorganisms, **characterized in that** carboxamides of the formula (I) according to Claim 1 are mixed with extenders and/or surfactants.

7. Use of carboxamides of the formula (I) according to Claim 1 for treating seed.

8. Use of carboxamides of the formula (I) according to Claim 1 for treating transgenic plants.

9. Use of carboxamides of the formula (I) according to Claim 1 for treating seed of transgenic plants.

10. Compounds of the formula (III-a) 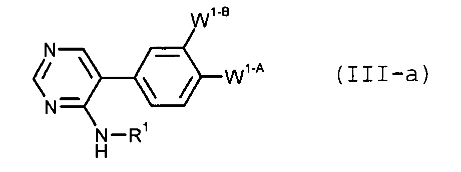 in which
R¹ is as defined in Claim 1,
and
W^{1-A} and W^{1-B} either both represent chlorine
or
W^{1-A} represents trifluoromethyl or trifluoromethoxy and W^{1-B} represents hydrogen.

## Revendications

1. Carboxamides de formule (I) 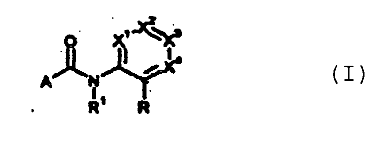 dans laquelle
R représente un groupe phényle éventuellement une à cinq fois substitué par W¹ ou représente le groupement 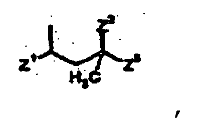
W¹ représente un atome d'halogène, un groupe cyano, nitro, alkyle en C₁-C₆, alcoxy en C₁-C₄, alkyl(C₁-C₄)thio, alkyl(C₁-C₄)sulfonyle, alcényle en C₂-C₆, cycloalkyle en C₃-C₆ ou halogénoalkyle(C₁-C₆) ; halogénoalcoxy(C₁-C₆), halogénoalkyl(C₁-C₆)thio ou halogénoalkyl(C₁-C₆)- sulfonyle comportant chacun de 1 à 13 atomes d'halogène, ou représente un groupe -C (Q²) =N-Q³, où,
Q² représente un atome d'hydrogène, un groupe hydroxy, alkyle en C₁-C₄, halogénoalkyle en C₁-C₄ comportant de 1 à 9 atomes d'halogène ou représente un groupe cycloalkyle en C₃-C₆,
Q³ représente un groupe hydroxy, amino, méthylamino, phényle, benzyle, ou représente un groupe alkyle en C₁-C₄ ou alcoxy en C₁-C₄ éventuellement substitués chacun par halogène, cyano, hydroxy, alcoxy en C₁-C₄, alkyl(C₁-C₄)thio, alkyl(C₁-C₄)amino, di[alkyl(C₁-C₄)]amino ou phényle ou représente un groupe alcényl(C₂-C₄)oxy ou alcynyl(C₂-C₄)oxy,
Z¹ représente un atome d'hydrogène ou le groupe méthyle,
Z² représente un atome d'hydrogène ou le groupe méthyle,
Z³ représente le groupe méthyle ou éthyle,
R¹ représente un atome d'hydrogène, un groupe alkyle en C₁-C₈, alkyl(C₁-C₆)sulfinyle, alkyl(C₁-C₆)- sulfonyle, alcoxy(C₁-C₄)-alkyle(C₁-C₄), cycloalkyle en C₃-C₈ ; halogénoalkyle (C₁-C₆), halogénoalkyl(C₁-C₄)thio, halogénoalkyl(C₁-C₄)- sulfinyle, halogénoalkyl(C₁-C₄)sulfonyle, halogéno- alcoxy(C₁-C₄)-alkyle(C₁-C₄), halogéno- cycloalkyle(C₃-C₈) comportant chacun de 1 à 9 atomes de fluor, de chlore et/ou de brome ; formyle, formyl-alkyle(C₁-C₃), [alkyl(C₁-C₃)]- carbonyl-alkyle(C₁-C₃), [alcoxy (C₁-C₃)]carbonyl- alkyle(C₁-C₃) ; halogéno-[alkyl(C₁-C₃)]carbonyl- alkyle(C₁-C₃), halogéno-[alcoxy(C₁-C₃)]carbonyl- alkyle(C₁-C₃) comportant chacun de 1 à 13 atomes de fluor, de chlore et/ou de brome ; alkyl(C₁-C₈)carbonyle, alcoxy(C₁-C₈)carbonyle, [alkylC₁-C₈)thio]carbonyle, [alcoxy(C₁-C₄)- alkyl(C₁-C₄)]carbonyle, [alcényl(C₃-C₆)oxy]- carbonyle, [alcynyl(C₃-C₆)oxy]carbonyle, [cycloalkyl(C₃-C₈)]carbonyle ; [halogénoalkyl(C₁-C₆)]carbonyle, [halogénoalcoxy(C₁-C₆)]carbonyle, [halogénoalkyl(C₁-C₆)thio]carbonyle, [halogénoalcoxy(C₁-C₄)-alkyl(C₁-C₄)]carbonyle, [halogénoalcényl(C₃-C₆)oxy]carbonyle, [halogénoalcynyl(C₃-C₆)oxy]carbonyle, [halogénocycloalkyl(C₃-C₈)]carbonyle comportant chacun de 1 à 9 atomes de fluor, de chlore et/ou de brome ; ou -CH₂-C≡C-R^{1-A}, -CH2-CH=CH-R^{1-A}, -CH=C=CH-R^{1-A}, -C (=O) C (=O) R², -CONR³R⁴ ou -CH₂NR⁵R⁶,
R^{1-A} représente un atome d'hydrogène, un groupe alkyle en C₁-C₆, halogénoalkyle(C₁-C₆), alcényle en C₂-C₆, alcynyle en C₂-C₆, cycloalkyle en C₃-C₇, [alcoxy(C₁-C₄)]carbonyle, [alcényl(C₃-C₆)oxy]- carbonyle, [alcynyl(C₃-C₆)oxy]carbonyle ou cyano,
R² représente un atome d'hydrogène, un groupe alkyle en C₁-C₈ ; alcoxy en C₁-C₈, alcoxy(C₁-C₄)- alkyle(C₁-C₄), cycloalkyle en C₃-C₈ ; halogénoalkyle(C₁-C₆), halogénoalcoxy(C₁-C₆), halogénoalcoxy(C₁-C₄)-alkyle(C₁-C₄), halogéno- cycloalkyle(C₃-C₈) comportant chacun de 1 à 9 atomes de fluor, de chlore et/ou de brome,
R³ et R⁴ représentent chacun, indépendamment l'un de l'autre, un atome d' hydrogène, un groupe alkyle en C₁-C₈, alcoxy(C₁-C₄)-alkyle(C₁-C₄), cycloalkyle en C₃-C₈ ; halogénoalkyle (C₁-C₈) , halogénoalcoxy(C₁-C₄)-alkyle(C₁-C₄), halogéno- cycloalkyle (C₃-C₈) comportant chacun de 1 à 9 atomes de fluor, de chlore et/ou de brome,
R³ et R⁴ en outre forment ensemble avec l'atome d'azote, auquel ils sont liés, un hétérocycle saturé ayant de 5 à 8 atomes formant le cycle, portant éventuellement un ou plusieurs substituants, identiques ou différents, halogène ou alkyle en C₁-C₄, l'hétérocycle pouvant contenir 1 ou 2 autres hétéroatomes non contigus, choisis dans l'ensemble constitué par les atomes d'oxygène, de soufre ou NR⁷,
R⁵ et R⁶ représentent, indépendamment l'un de l'autre, un atome d'hydrogène, un groupe alkyle en C₁-C₈, cycloalkyle en C₃-C₈, halogénoalkyle en C₁-C₈, halogénocycloalkyle en C₃-C₈ comportant chacun de 1 à 9 atomes de fluor, de chlore et/ou de brome,
R⁵ et R⁶ en outre forment ensemble avec l'atome d'azote, auquel ils sont liés, un hétérocycle saturé ayant de 5 à 8 atomes formant le cycle, portant éventuellement un ou plusieurs substituants, identiques ou différents, halogène ou alkyle en C₁-C₄, l'hétérocycle pouvant contenir 1 ou 2 autres hétéroatomes con contigus, choisis dans l'ensemble constitué par les atomes d'oxygène, de soufre ou NR⁷,
R⁷ représente un atome d'hydrogène ou un groupe alkyle en C₁-C₆,
X¹, X², X³ et X⁴ représentent, indépendamment les uns des autres, N ou CR⁸, étant entendu qu'au moins l'un de ces radicaux représente N,
R⁸ représente un atome d'hydrogène, de fluor, de chlore, le groupe méthyle, isopropyle, méthylthio ou trifluorométhyle,
A représente l'un des radicaux A1 à A18 suivants 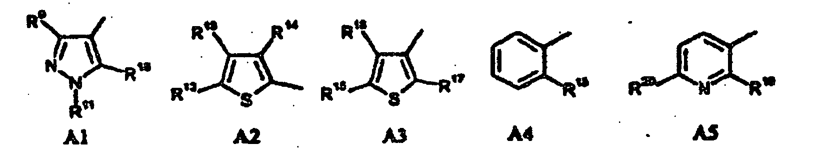 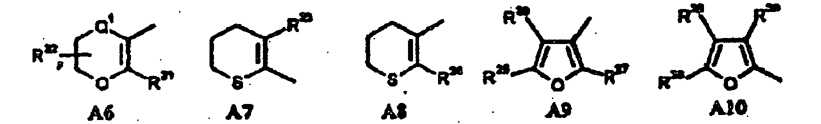 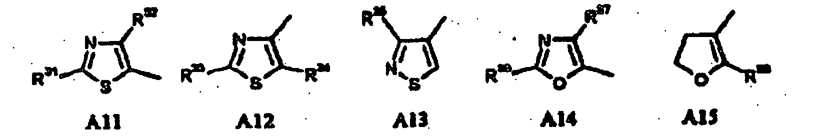 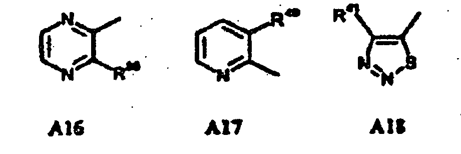
R⁹ représente un atome d'hydrogène ou d'halogène, un groupe cyano, nitro, alkyle en C₁-C₄, alcoxy en C₁-C₄, alkyl(C₁-C₄)thio, cycloalkyle en C₃-C₆, halogénoalkyle(C₁-C₄), halogénoalcoxy(C₁-C₄) ou halogénoalkyl(C₁-C₄)thio comportant chacun de 1 à 5 atomes d'halogène, un groupe aminocarbonyle ou aminocarbonyl-alkyle(C₁-C₄),
R¹⁰ représente un atome d'hydrogène ou d'halogène, un groupe cyano, alkyle en C₁-C₄, alcoxy en C₁-C₄ ou alkyl(C₁-C₄)thio,
R¹¹ représente un atome d'hydrogène, un groupe alkyle en C₁-C₄, hydroxyalkyle(C₁-C₄), alcényle en C₂-C₆, cycloalkyle en C₃-C₆, alkyl(C₁-C₄)thio- alkyle(C₁-C₄), alcoxy(C₁-C₄)-alkyle(C₁-C₄), halogénoalkyle (C₁-C₄), halogénoalkyl(C₁-C₄)thio- alkyle(C₁-C₄), halogénoalcoxy(C₁-C₄)-alkyle(C₁-C₄) comportant chacun de 1 à 5 atomes d'halogène, phényle, [alkyl(C₁-C₄)]carbonyle, [alcoxy(C₁-C₄)]- carbonyle, [alkyl(C₁-C₄)thio]carbonyle, [alcoxy(C₁-C₄)-alkyl(C₁-C₄)]carbonyle ;
[halogénoalkyl(C₁-C₄)]carbonyle, [halogénoalkyl(C₁-C₄)thio]carbonyle, [halogéno- alcoxy(C₁-C₄)-alkyl(C₁-C₄)]carbonyle, comportant chacun de 1 à 9 atomes d'halogène,
R¹² et R¹³ représentent, indépendamment l'un de l'autre, un atome d'hydrogène ou d'halogène, un groupe alkyle en C₁-C₄ ou halogénoalkyle(C₁-C₄) comportant de 1 à 5 atomes d'halogène,
R¹⁴ représente un atome d'halogène, un groupe cyano ou alkyle en C₁-C₄, ou halogénoalkyle(C₁-C₄) ou halogénoalcoxy(C₁-C₄) comportant chacun de 1 à 5 atomes d'halogène,
R¹⁵ et R¹⁶ représentent, indépendamment l'un de l'autre, un atome d'hydrogène ou d'halogène, un groupe alkyle en C₁-C₄ ou halogénoalkyle(C₁-C₄) comportant de 1 à 5 atomes d'halogène,
R¹⁷ représente un atome d'hydrogène, un groupe alkyle en C₁-C₄ ou halogénoalkyle (C₁-C₄) comportant de 1 à 5 atomes d'halogène,
R¹⁸ représente un atome d'hydrogène ou d'halogène, un groupe hydroxy, cyano, alkyle en C₁-C₆, halogénoalkyle(C₁-C₄), halogénoalcoxy(C₁-C₄) ou halogénoalkyl(C₁-C₄)thio comportant chacun de 1 à 5 atomes d'halogène,
R¹⁹ représente un atome d' halogène, un groupe hydroxy, cyano, alkyle en C₁-C₄, alcoxy en C₁-C₄, alkyl(C₁-C₄)thio, halogénoalkyle(C₁-C₄), halogénoalkyl(C₁-C₄)thio ou halogénoalcoxy(C₁-C₄) comportant chacun de 1 à 5 atomes d'halogène,
R²⁰ représente un atome d'hydrogène ou d'halogène, un groupe cyano, alkyle en C₁-C₄, alcoxy en C₁-C₄, alkyl(C₁-C₄)thio, halogénoalkyle(C₁-C₄), halogénoalcoxy(C₁-C₄) comportant chacun de 1 à 5 atomes d'halogène, alkyl(C₁-C₄)sulfinyle ou alkyl(C₁C₄)sulfonyle,
R²¹ représente un groupe alkyle en C₁-C₄ ou halogénoalkyle(C₁-C₄) comportant de 1 à 5 atomes d'halogène,
R²² représente un groupe alkyle en C₁-C₄,
Q¹ représente S (un atome de soufre), SO, SO₂ ou CH₂,
p représente 0, 1 ou 2, lorsque p représente 2 R²² représentant des radicaux identiques ou différents,
R²³ représente un groupe alkyle en C₁-C₄ ou halogénoalkyle(C₁-C₄) comportant de 1 à 5 atomes d'halogène,
R²⁴ représente un groupe alkyle en C₁-C₄ ou halogénoalkyle(C₁-C₄) comportant de 1 à 5 atomes d'halogène,
R²⁵ et R²⁶ représentent, indépendamment l'un de l'autre, un atome d'hydrogène ou d'halogène, un groupe amino, alkyle en C₁-C₄ ou halogénoalkyle(C₁-C₄) comportant de 1 à 5 atomes d'halogène,
R²⁷ représente un atome d'hydrogène, un groupe alkyle en C₁-C₄ ou halogénoalkyle(C₁-C₄) comportant de 1 à 5 atomes d'halogène,
R²⁸ et R²⁹ représentent, indépendamment l'un de l'autre, un atome d'hydrogène ou d'halogène, un groupe amino, nitro, alkyle en C₁-C₄ ou halogénoalkyle(C₁-C₄) comportant de 1 à 5 atomes d'halogène,
R³⁰ représente un atome d'hydrogène ou d'halogène, un groupe alkyle en C₁-C₄ ou halogénoalkyle(C₁-C₄) comportant de 1 à 5 atomes d'halogène,
R³¹ représente un atome d'hydrogène ou d'halogène, un groupe amino, alkyl(C₁-C₄)amino, di[alkyl(C₁-C₄)]-amino, cyano, alkyle en C₁-C₄ ou halogénoalkyle(C₁-C₄) comportant de 1 à 5 atomes d'halogène,
R³² représente un atome d'halogène, un groupe alkyle en C₁-C₄ ou halogénoalkyle(C₁-C₄) comportant de 1 à 5 atomes d'halogène,
R³³ représente un atome d'hydrogène ou d'halogène, un groupe amino, alkyl(C₁-C₄)amino, di[alkyl(C₁-C₄)]- amino, cyano, alkyle en C₁-C₄ ou halogénoalkyle(C₁-C₄) comportant de 1 à 5 atomes d'halogène,
R³⁴ représente un atome d'halogène, un groupe alkyle en C₁-C₄ ou halogénoalkyle(C₁-C₄) comportant de 1 à 5 atomes d'halogène,
R³⁵ représente un atome d'halogène, un groupe alkyle en C₁-C₄ ou halogénoalkyle(C₁-C₄) comportant de 1 à 5 atomes d'halogène,
R³⁶ représente un atome d'hydrogène ou un groupe alkyle en C₁-C₄,
R³⁷ représente un atome d'halogène ou un groupe alkyle en C₁-C₄,
R³⁸ représente un groupe alkyle en C₁-C₄ ou halogénoalkyle(C₁-C₄) comportant de 1 à 5 atomes d'halogène,
R³⁹ représente un atome d'hydrogène ou d'halogène, un groupe alkyle en C₁-C₄ ou halogénoalkyle(C₁-C₄) comportant de 1 à 5 atomes d'halogène,
R⁴⁰ représente un atome d'halogène, un groupe hydroxy, alkyle en C₁-C₄, alcoxy en C₁-C₄, alkyl(C₁-C₄)thio, halogénoalkyle(C₁-C₄), halogénoalkyl(C₁-C₄)thio ou halogénoalcoxy(C₁-C₄) comportant chacun de 1 à 5 atomes d'halogène,
R⁴¹ représente un groupe alkyle en C₁-C₄.

2. Procédé pour la préparation de carboxamides de formule (I) selon la revendication 1, **caractérisé en ce que**
(a) on fait réagir des halogénures de carbonyle de formule (II) 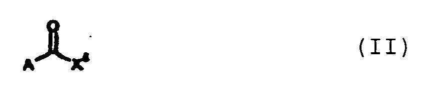 dans laquelle
A a les significations données dans la revendication 1,
X⁵ représente un atome d'halogène ou le groupe hydroxy, avec des dérivés d'aniline de formule (III) 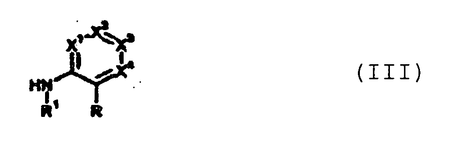 dans laquelle
R, R¹, X¹, X², X³ et X⁴ ont les significations données dans la revendication 1, éventuellement en présence d'un réactif de couplage, éventuellement en présence d'un capteur d'acide et éventuellement en présence d'un diluant,
ou
(b) on fait réagir des carboxamides de formule (I-a) 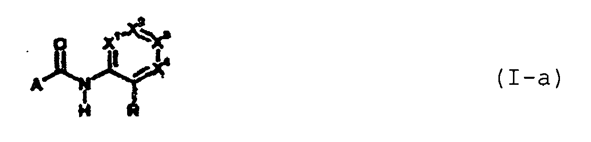 dans laquelle R, X¹, X², X³, X⁴ et A ont les significations données dans la revendication 1, avec des halogénures de formule (IV)
R^{1-B}-Hal (IV)
dans laquelle
R^{1-B} représente un groupe alkyle en C₁-C₈, alkyl(C₁-C₆)sulfinyle, alkyl(C₁-C₆)sulfonyle, alcoxy(C₁-C₄)-alkyle(C₁-C₄), cycloalkyle en C₃-C₈ ; halogénoalkyle (C₁-C₆), halogénoalkyl(C₁-C₄)thio, halogénoalkyl(C₁-C₄)- sulfinyle, halogénoalkyl(C₁-C₄)sulfonyle, halogéno-alcoxy(C₁-C₄)-alkyle(C₁-C₄), halogéno- cycloalkyle(C₃-C₈) comportant chacun de 1 à 9 atomes de fluor, de chlore et/ou de brome ; formyle, formyl-alkyle(C₁-C₃), [alkyl(C₁-C₃)]- carbonyl-alkyle(C₁-C₃), [alcoxy(C₁-C₃)]carbonyl-alkyle(C₁-C₃); halogéno-[alkyl(C₁-C₃)]carbonyl-alkyle(C₁-C₃), halogéno-[alcoxy(C₁-C₃)]carbonyl-alkyle(C₁-C₃) comportant chacun de 1 à 13 atomes de fluor, de chlore et/ou de brome ; alkyl(C₁-C₈)carbonyle, alcoxy(C₁-C₈)carbonyle, [alkyl(C₁-C₈)thio]carbonyle, [alcoxy(C₁-C₄)- alkyl(C₁-C₄)]carbonyle, [alcényl(C₃-C₆)oxy]- carbonyle, [alcynyl(C₃-C₆)oxy]carbonyle, [cycloalkyl(C₃-C₈)]carbonyle ; [halogénoalkyl(C₁-C₆)]carbonyle, [halogénoalcoxy(C₁-C₆)]carbonyle, [halogénoalkyl(C₁-C₆)thio]carbonyle, [halogénoalcoxy(C₁-C₄)-alkyl(C₁-C₄)]carbonyle, [halogénoalcényl(C₃-C₆)oxy]carbonyle, [halogénoalcynyl(C₃-C₆)oxy]carbonyle, [halogénocycloalkyl(C₃-C₈)]carbonyle comportant chacun de 1 à 9 atomes de fluor, de chlore et/ou de brome ou -CH₂-C≡C-R^{1-A}, -CH₂-CH=CH-R^{1-A}, -CH=C=CH-R^{1-A}, -C(=O)C(=O)R², -CONR³R⁴ ou -CH₂NR⁵R⁶,
R^{1-A}, R², R³, R⁴, R⁵ et R⁶ ont les significations données dans la revendication 1,
Hal représente un atome de chlore, de brome ou d'iode,
en présence d'une base et en présence d'un diluant.

3. Composition pour la lutte contre des micro-organismes indésirables, **caractérisée par** une teneur en au moins un carboxamide de formule (I) selon la revendication 1, en plus d'excipients et/ou de substances tensioactives.

4. Utilisation de carboxamides de formule (I) selon la revendication 1, pour la lutte contre des micro-organismes indésirables dans la protection de plantes et la protection de matériaux.

5. Procédé pour la lutte contre des micro-organismes indésirables dans la protection de plantes et la protection de matériaux, **caractérisé en ce qu'**on applique sur les micro-organismes ou et/ou leur habitat des carboxamides de formule (I) selon la revendication 1.

6. Procédé pour la préparation de compositions destinées à la lutte contre des micro-organismes indésirables, **caractérisé en ce qu'**on mélange des carboxamides de formule (I) selon la revendication 1 avec des excipients et/ou des substances tensioactives.

7. Utilisation de carboxamides de formule (I) selon la revendication 1, pour le traitement de semences.

8. Utilisation de carboxamides de formule (I) selon la revendication 1, pour le traitement de plantes transgéniques.

9. Utilisation de carboxamides de formule (I) selon la revendication 1, pour le traitement de semences de plantes transgéniques.

10. Composés de formule (III-a) 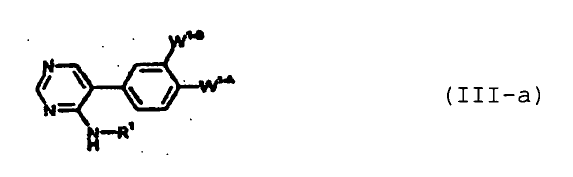 dans laquelle
R¹ a les significations données dans la revendication 1,
et
soit W^{1-A} et W^{1-B} représentent en même temps un atome de chlore,
soit W^{1-A} représente le groupe trifluorométhyle et W^{1-B} représente un atome d'hydrogène.
